# EUROPEAN PATENT SPECIFICATION

(11) **EP 4 076 310 B1**
(45) Date of publication and mention of the grant of the patent: **06.03.2024**
(21) Application number: 20828754.0
(22) Date of filing: 18.12.2020
(51) Int. Cl.: A61F 13/00

(54) **WOUND DRESSING SYSTEM**
WUNDVERBANDSYSTEM
SYSTÈME DE PANSEMENT

(30) Priority: 18.12.2019 DK PA201970788
(43) Date of publication of application: 26.10.2022
(73) Proprietor: Coloplast A/S, 3050 Humlebaek (DK)
(72) Inventor: NIELSEN, Finn, 2300 Copenhagen S (DK); IGWEBUIKE, Henning, 3540 Lynge (DK); HOEIER NIELSEN, Kent, 3650 Oelstykke (DK)
(86) International application number: PCT/DK2020/050383
(87) International publication number: WO 2021/121518

(56) References cited:
- US-A1- 2012 190 956

## Description

The present disclosure relates to a wound dressing and in particular to a wound dressing with sensing capability to facilitate monitoring of the wound/wound dressing during use of the wound dressing. A wound dressing system, and devices of the wound dressing system are also disclosed. The wound dressing system comprises a wound dressing and a monitor device. In particular, the present disclosure relates to a monitor device configured to determine an exudate uptake pattern of a wound dressing.

### Brief description of the drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates a perspective view of a model of a wound dressing for a wound dressing system according to an embodiment of the invention,
Fig. 2A illustrates a top view of a model of a wound dressing for a wound dressing system according to an embodiment of the invention,
Fig. 2B illustrates a cross-sectional view of a model of a wound dressing for a wound dressing system according to an embodiment of the invention,
Fig. 3A illustrates a top view of a model of a wound dressing for a wound dressing system according to an embodiment of the invention,
Fig. 3B illustrates a top view of a model of a wound dressing for a wound dressing system according to an embodiment of the invention,
Fig. 4 illustrates a top view of a model of a wound dressing for a wound dressing system according to an embodiment of the invention,
Fig. 5A illustrates a top view of a distal electrode assembly, a proximal electrode assembly, and a perforated spacer element for a wound dressing according to an embodiment of the invention,
Fig. 5B illustrates a cross-sectional view of a distal electrode assembly, a proximal electrode assembly, and a perforated spacer element for a wound dressing according to an embodiment of the invention,
Fig. 6 illustrates a cross-sectional view of a wound dressing for a wound dressing system according to an embodiment of the invention,
Fig. 7A illustrates an arrangement of electrodes of a proximal and distal electrode assembly according to an embodiment of the invention,
Fig. 7B illustrates an arrangement of electrodes of a proximal and distal electrode assembly according to an embodiment of the invention,
Fig. 8 illustrates an exploded view of a sensor pack for a wound dressing of a wound dressing system according to an embodiment of the invention,
Fig. 9 illustrates a close-up view of a sensor pack for a wound dressing of a wound dressing system according to an embodiment of the invention,
Fig. 10 illustrates an exploded view of a wound dressing system according to an embodiment of the invention,
Fig. 11 illustrates a schematic block diagram of an exemplary monitor device according to an embodiment of the invention, and
Fig. 12 illustrates an exemplary wound dressing system according to an embodiment of the invention.

### Background

US 2012/190956 A1 relates to a wound sensor comprising at least one electrode and a non-adherent porous layer proximate at least part of the electrode.

### Detailed description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

In the following, whenever referring to proximal side or surface of a layer, an element, a device or part of a device, the referral is to the skin-facing side or surface, when a user wears the wound dressing. Likewise, whenever referring to the distal side or surface of a layer, an element, a device or part of a device, the referral is to the side or surface facing away from the skin, when a user wears the wound dressing. In other words, the proximal side or surface is the side or surface closest to the user, when the wound dressing is fitted on a user and the distal side is the opposite side or surface - the side or surface furthest away from the user in use.

The normal direction is defined as the direction being normal to the skin surface of the user, when a user wears the wound dressing. Thus, the normal direction is generally perpendicular to the skin surface of the user when the user wears the wound dressing. Generally, the wound dressing is substantially planar (has a planar extension), such that a major planar surface of the wound dressing can be arranged parallel to the skin, e.g. adhered to the skin. Thus, the normal direction is generally normal to the planar extension of the wound dressing. The planar extension of the wound dressing may be denoted the appliance plane.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure provides a wound dressing system comprising a wound dressing and a monitor device, a wound dressing, and a method for predicting an exudate uptake pattern.

The invention describes a wound dressing system configured to predict an exudate uptake pattern of a wound dressing. The wound dressing system comprises a wound dressing and a monitor device. The wound dressing extends in an appliance plane and comprises an absorbent core layer having a proximal side and distal side, a proximal electrode assembly comprising a first plurality of electrodes, and a distal electrode assembly comprising a second plurality of electrodes. The proximal electrode assembly and the distal electrode assembly are layered and spaced apart by a first gap in a direction normal to the appliance plane. The monitor device is configured to detect a short-circuiting event across a primary and a secondary electrode of the first plurality of electrodes and to detect a short-circuiting event across a primary and secondary electrode of the second plurality of electrodes. Further, the monitor device is configured to determine a first region of the wound dressing, wherein the first region is a region where the short-circuited primary and secondary electrodes of the first plurality of electrodes and the short-circuited primary and secondary electrodes of the second plurality of electrodes overlap in the direction normal to the appliance plane (in the following; the normal direction), and to indicate an exudate uptake pattern of the wound dressing based on at least the first region.

The present disclosure relates to a wound dressing system and devices thereof, such as a wound dressing, a monitor device, and optionally one or more accessory devices. Further, methods related to the wound dressing system and devices thereof are disclosed. An accessory device (also referred to as an external device) can be a mobile phone, smartphone, or other handheld device. An accessory device can be a personal electronic device, e.g. a wearable, such as a watch or other wrist-worn electronic device. In embodiments, an accessory device is a docking station. In embodiments, the docking station is configured to electrically and/or mechanically couple the monitor device to the docking station. In embodiments, the docking station is configured for charging the monitor device and/or configured for transferring data between the monitor device and the docking station. In embodiments, the wound dressing system comprises a server device. The server device can be operated and/or controlled by the wound dressing system manufacturer and/or a service centre.

The present disclosure provides a wound dressing system and devices thereof, such as a wound dressing, a monitor device, and optionally one or more accessory devices which either alone or together facilitate reliable monitoring of the wound dressing and operating state thereof. Accordingly, the wound dressing system and devices thereof enable providing information to the user about the operating state (such as specific or different exudate uptake patterns, and/or exudate uptake rates) of the wound dressing, and in turn optionally enable providing an indication to the user or a caretaker of the remaining time frame for replacing the wound dressing, such as to provide optimum wound healing conditions.

The wound dressing comprises an absorbent core layer and a proximal and a distal electrode assembly. In embodiments the wound dressing further comprises a first adhesive layer arranged on a proximal side of the absorbent core layer, the first adhesive layer having a proximal surface configured for attachment of the wound dressing to the skin surface of a user. In embodiments, the wound dressing comprises a monitor interface configured for electrically and/or mechanically facilitate coupling to a monitor device. In embodiments, the electrode assemblies and the monitor interface are provided in a separate sensor pack layered with the wound dressing (absorbent core layer), thereby allowing an efficient manufacturing process, wherein the sensor pack can be assembled separately. In the following, reference is made to a wound dressing comprising the electrode assemblies, but the referral may as well be substituted with a referral to an absorbent core layer layered with a sensor pack comprising the electrode assemblies. Thus, in embodiments, the wound dressing of the wound dressing system comprises an absorbent core layer having a proximal side and a distal side, and a sensor pack comprising a proximal electrode assembly comprising a first plurality of electrodes, a distal electrode assembly comprising a second plurality of electrodes, and a spacer element providing a first gap between said assemblies (i.e., in a direction normal to the appliance plane).

It is an advantage of the present disclosure that an optimum or improved use of a wound dressing is enabled and facilitated. In particular, the present disclosure facilitates that a wound dressing is not changed too early (leading to increased costs and/or material waste) nor too late (leading to adhesive failure, leakage, and/or unsatisfactory wound healing conditions). Accordingly, the user or a health care professional is able to monitor and plan the use of the wound dressing. Determination of moisture or wetting patterns, such as exudate uptake patterns, and/or wetting types and/or classification of operating states of the wound dressing is useful in helping to reduce the risk of a user experiencing leakage from a wound dressing and/or in helping reduce the risk of unsatisfactory wound healing conditions. The present disclosure provides an efficient and easy-to-use wound dressing system with a high degree of comfort for a user.

In embodiments, the wound dressing of the wound dressing system comprises a first adhesive layer having a proximal surface configured for attachment of the wound dressing to the skin surface of a user. The first adhesive layer can comprise or be made of a first composition. In embodiments, the first composition comprises silicone. In embodiments, the first adhesive layer comprises a support layer with an adhesive material made of a first composition moulded onto or otherwise attached to the support layer. In embodiments, the first composition is a thermoset, curable adhesive material. An example of such adhesive material can be a silicone based adhesive material. In embodiments, the first composition is a two-component system. Preferably, the first composition contains no solvent. Preferred first compositions include polyurethane, acrylic, silicone or polyethylene or polypropylene oxide based cross-linking types, e.g. as described in WO 2005/032401. In embodiments, the first composition is a hotmelt type, which initially is heated to flow and subsequently cooled to gel or crosslink. In embodiments, instead of curing upon cooling, the first composition can in some embodiments cure upon application of thermal energy. In embodiments, the support layer of the first adhesive layer is any suitable layer being water impermeable but vapour permeable. A suitable support layer can be a polyurethane film.

In embodiments, the first adhesive layer of the wound dressing of the wound dressing system has perforations or through-going openings arranged within an absorbing region, e.g. for allowing exudate from the wound to pass or flow through the perforations of the first adhesive layer to be absorbed by the absorbent core layer arranged on the distal side of the first adhesive layer. In embodiments, the perforations of the first adhesive layer are made by punching, cutting, or by applying high frequency mechanical vibrations, for example as disclosed in WO 2010/061228. In embodiments, the perforations are arranged in a regular or random array, typically separated by 0.5 mm to 10 mm. The number of holes per cm2 may be between 1 and 100, such as between 1 and 50, or even between 2 and 20. In embodiments, the perforations of the first adhesive layer have a diameter in the range from 0.5 mm to 10 mm, such as in the range from 1 mm to 8 mm. In one or more exemplary wound dressings, the perforations of the first adhesive layer have a diameter in the range from 1 mm to 5 mm, e.g. from 1.5 mm to 5 mm, and even in the range from 2 mm to 4 mm.

The wound dressing of the wound dressing system comprises an absorbent core layer also denoted an absorbent pad. In embodiments, the absorbent core layer is a uniform material or a composite, for example in the form of a layered construction comprising layers of different texture and properties. In embodiments, the absorbent core layer comprises foam, cellulose, super absorbent particles, and/or fibres. In embodiments, the absorbent core layer comprises a layer of foam facing the wound. In embodiments, the absorbent core layer comprises a polyurethane foam. In embodiments, the absorbent core layer comprises a super absorbing layer. In embodiments, the absorbent core layer can comprise one or more polyisobutenes and/or styrene-isoprene-styrene. In embodiments, absorbent core layer comprises one or more hydrocolloids, such as one or more water soluble or water swellable hydrocolloids. The absorbent core layer can be a pressure sensitive adhesive composition suitable for medical purposes comprising a rubbery elastomeric base. In embodiments, the absorbent core layer comprises an adhesive, such as known from adhesive hydrocolloid-comprising layers, whereby the absorbent core layer can be adhered directly to the skin/wound. In embodiments, the absorbent core layer and/or the first adhesive layer contains active ingredients, such as ibuprofen, paracetamol, silver compounds, or other medically active ingredients and is/are configured to release the active ingredients to the wound to reduce pain and/or to improve the healing of the wound. In one or more exemplary wound dressings, the absorbent core layer comprises a silver compound with antimicrobial properties.

In embodiments, the wound dressing of the wound dressing system comprises a top layer also denoted a backing layer or a top film. In embodiments, the top layer is a distal-most component of the wound dressing. In embodiments, the top layer can be any suitable layer being water impermeable but vapour permeable. In embodiments, a suitable top layer is a polyurethane film. In embodiments, the top layer is provided in the aforementioned sensor pack layered with the absorbent core layer of the wound dressing. The top layer is a protective layer protecting the absorbent core layer and other parts of the wound dressing from external strains and stress when the user wears the wound dressing. In embodiments, the top layer has a thickness in the range from 0.01 mm to 1.0 mm, e.g. in the range from 0.02 mm to 0.2 mm, such as 0.04 mm.

In embodiments, the top layer comprises a top layer opening configured to allow for connection between the plurality of electrodes of the electrode assemblies and terminals of the monitor device, of the wound dressing system, coupled to the wound dressing. For example, the top layer opening can allow for a first part of the electrode assemblies to extend through the top layer to form a part of a monitor interface of the wound dressing.

In embodiments, the wound dressing of the wound dressing system comprises a release liner, such as a one-piece, two-piece, or a three-piece release liner. The release liner is a protective layer that protects adhesive layer(s) during transport and storage and is peeled off by the user prior to applying the wound dressing on the skin.

The wound dressing (or sensor pack) of the wound dressing system comprises a proximal electrode assembly comprising a first plurality of electrodes and a distal electrode assembly comprising a second plurality of electrodes. The electrodes are electrically conductive and can comprise one or more of metallic (e.g. silver, copper, gold, titanium, aluminium, stainless steel), ceramic (e.g. ITO), polymeric (e.g. PEDOT, PANI, PPy), and carbonaceous (e.g. carbon black, carbon nanotube, carbon fibre, graphene, graphite) materials. In embodiments, the electrodes are in the form of electrodes provided, such as printed, on a support layer, such as a flexible and/or polymeric layer. In embodiments, the electrodes are in the form of wires. For example, a wire can be a string-like piece of relatively rigid or flexible conductive material, e.g. metal, thereby having a certain degree of structural integrity.

In embodiments, each electrode comprises a connection part, e.g. to form a terminal of the monitor interface or for connecting the respective electrode to other components and/or to interface terminals/terminal elements. Thus, in embodiments, a connection part forms a terminal allowing a corresponding interface of a monitor device to be electrically coupled to the electrode(s).

The proximal and distal electrode assemblies are layered, such that they form a layered/sandwiched structure. The proximal and distal electrode assemblies are spaced apart by a first gap in a direction normal to the appliance plane of the wound dressing (or of the sensor pack). In embodiments, the proximal and distal electrode assemblies are spaced apart by a first material, such that said first material forms the first gap between the two electrode assemblies. The gap serves to avoid short-circuits to form across/between the assemblies (inter-short-circuits) in an initial configuration (before use/wetting of the wound dressing). For example, the first gap is provided by arranging the proximal electrode assembly on a proximal side or surface of the absorbent core layer, and by arranging the distal electrode assembly on a distal side or surface of the absorbent core layer. Thereby, the first material may be the absorbent core layer, or at least parts thereof. Alternatively, the first gap can be provided by arranging the electrode assemblies on each side or surface of a (planar) spacer element, such as a spacer element arranged on a distal or a proximal side of the absorbent core layer, or such as a spacer element of a sensor pack. Thereby, the first material may be the spacer element. In embodiments, the spacer element is the support layer on which the electrodes are provided. In embodiments, the spacer element comprises a polymeric film, such as a (thermoplastic) polyurethane film. In embodiments, the proximal and distal electrode assemblies are planar electrode assemblies spanning a certain geometrical plane, such parallel with the appliance plane. In embodiments, the proximal electrode assembly spans a first geometrical plane and the distal electrode assembly spans a second geometrical plane. In embodiments, the first geometrical plane and the second geometrical plane are parallel and non-coinciding (namely; spaced apart by the first gap/material). In embodiments, the first material has a constant distance separating the proximal electrode assembly from the distal electrode assembly. That is, in embodiments, the first gap is a (constant) distance separating the proximal electrode assembly from the distal electrode assembly. In embodiments, the size of separation is dependent on a thickness of the first material, such as the absorbent core layer or the spacer element of the wound dressing/sensor pack. In embodiments, the first material is an (at least initially) insulative material, such as the absorbent core layer or a spacer element. In embodiments, the first material has a thickness less than 10 mm, such as less than 5 mm. In embodiments, the first material has a size/width/thickness less than 100 µm, such as embodied by a polymeric film, thereby allowing for a fast transfer of exudate/liquid from the proximal electrode assembly to the distal electrode assembly. In embodiments, the first material has a thickness between 30 µm and 100 µm. In embodiments, the first material is the absorbent core layer, whereby the thickness of the first material can be between 0.8 mm and 10 mm, such as 0.8 mm, or 1 mm, or 2 mm, or 3 mm, or 4 mm, or 5 mm, or 6 mm, or 7 mm, or 8 mm, or 9 mm, or 10 mm.

The proximal electrode assembly comprises a first plurality of electrodes, such as two or more electrodes, such as 2, 3, 4, 5, 6, 7, 8, 9, or 10 electrodes, or such as between 10 and 20 electrodes, or such as more than 20 electrodes. In embodiments, the number of electrodes is selected dependent on the size of the wound dressing as such, such as a number providing a certain fixed distance between the electrodes. In embodiments the electrodes forming the first plurality of electrodes are mutually parallel and/or equally spaced by a first distance. In embodiments, the first distance is less than 10 mm, such as less than 8 mm, such as less than 6 mm, such as 4 mm or 2 mm. In embodiments, the first distance is larger than 10 mm, such as between 10 mm and 50 mm, such as between 15 mm and 30 mm. Providing a large first distance can be beneficial when the wound dressing is particularly large, such as 20 cm or more along a certain direction. In embodiments, the proximal electrode assembly covers at least a part of a surface of the absorbent core layer, such as the entire surface of the absorbent core layer. Thereby, the proximal electrode assembly can be used for monitoring the wound dressing, in particular any wetting, e.g. due to exudate uptake in the absorbent core layer.

The distal electrode assembly comprises a second plurality of electrodes, such as two or more electrodes, such as 2, 3, 4, 5, 6, 7, 8, 9, or 10 electrodes, or such as between 10 and 20 electrodes, or such as more than 20 electrodes. In embodiments, the number of electrodes is selected dependent on the size of the wound dressing as such, such as a number providing a certain fixed distance between the electrodes. In embodiments the electrodes forming the second plurality of electrodes are mutually parallel and/or equally spaced by a second distance. In embodiments, the second distance is less than 10 mm, such as less than 8 mm, such as less than 6 mm, such as 4 mm or 2 mm. In embodiments, the second distance is larger than 10 mm, such as between 10 mm and 50 mm, such as between 15 mm and 30 mm. Providing a large second distance can be beneficial when the wound dressing is particularly large, such as 20 cm or more along a certain direction. In embodiments, the distal electrode assembly covers at least a part of a surface of the absorbent core layer, such as the entire surface of the absorbent core layer. Thereby, the distal electrode assembly can be used for monitoring the wound dressing, in particular any wetting, e.g. due to exudate uptake in the absorbent core layer.

In embodiments, the first plurality of electrodes and the second plurality of electrodes comprises an equal/identical number of electrodes. In embodiments, the first plurality of electrodes and the second plurality of electrodes comprises an unequal/non-identical number of electrodes. In embodiments, the first distance and the second distance between the electrodes of the first/second plurality of electrodes are identical. In embodiments, the proximal electrode assembly is arranged proximal to the distal electrode assembly, such that the proximal electrode assembly is closer to the skin surface of the user than the distal electrode assembly when the wound dressing is being worn by a user. In embodiments, the proximal electrode assembly is arranged on a proximal side of the absorbent core layer, such as on the proximal surface of the absorbent core layer. In embodiments, the distal electrode assembly is arranged on a distal side of the absorbent core layer, such as on the distal surface of the absorbent core layer.

In embodiments, the geometrical plane of the proximal electrode assembly is parallel with the geometrical plane of the distal electrode assembly, such that the assemblies can be considered layered. Further, in embodiments, the electrodes of the first plurality of electrodes cross/intersect the electrodes of the second plurality of electrodes, i.e. forms an array, when viewed in a direction normal to the geometrical planes of the electrode assemblies. In other words, the electrodes of the first plurality of electrodes are oriented non-parallel to the electrodes of the second plurality of electrodes. In embodiments, the electrodes of the proximal electrode assembly are rotated by an angle selected between 45 degrees and 135 degrees, such as 90 degrees, relative to the electrodes of the distal electrode assembly.

In embodiments, the proximal electrode assembly comprises one or more support layers including a first support layer. In embodiments, the first support layer comprises perforations, e.g. for allowing exudate to pass through the first support layer. In embodiments, the perforations in the first support layer are not aligned/coinciding with electrodes of the proximal electrode assembly such that exudate can pass through the support layer. In embodiments, the first support layer is fluid-permeable. In embodiments, the first plurality of electrodes is printed or arranged on a surface of the first support layer. In embodiments, the first plurality of electrodes is printed or arranged on a proximal surface of the first support layer. In embodiments, the first support layer is the spacer element, such as the spacer element of a sensor pack.

In embodiments, the distal electrode assembly comprises one or more support layers including a second support layer. In embodiments, the second support layer comprises perforations, e.g. for allowing exudate to pass through the second support layer. In embodiments, the perforations in the second support layer are not aligned/coinciding with electrodes of the distal electrode assembly such that exudate can pass through the support layer. In embodiments, the second support layer is fluid-permeable. In embodiments, the second plurality of electrodes is printed or arranged on a surface of the second support layer. In embodiments, the second plurality of electrodes is printed or arranged on a distal surface of the second support layer. In embodiments, the second support layer is the spacer element, such as the spacer element of a sensor pack. In embodiments, the second support layer is the first support layer, such that the proximal electrode assembly may be arranged on the proximal side of the first support layer and the distal electrode assembly may be arranged on the distal side of the first support layer.

In embodiments, a support layer, such as the first support layer and/or the second support layer, comprises polymeric (e.g. polyurethane, PTFE, PVDF) and/or ceramic (e.g. alumina, silica) materials. In embodiments, the first support layer and/or the second support layer is/are made of thermoplastic polyurethane (TPU). In embodiments, the support layer material is made of or comprises one or more of polyester, a thermoplastic elastomer (TPE), polyamide, polyimide, Ethylene-vinyl acetate (EVA), polyurea, and silicones. Exemplary thermoplastic elastomers of the first support layer and/or the second support layer are styrenic block copolymers (TPS, TPE-s), thermoplastic polyolefin elastomers (TPO, TPE-o), thermoplastic Vulcanizates (TPV, TPE-v), thermoplastic polyurethanes (TPU), thermoplastic copolyester (TPC, TPE-E), and thermoplastic polyamides (TPA, TPE-A).

In embodiments, the proximal and/or the distal electrode assembly comprises a reinforcement element. In embodiments, the reinforcement element forms part of at least a part of the electrode assemblies. The reinforcement element optionally comprises a plurality of conductive paths respectively connected to electrodes of the first and/or second plurality of electrodes. In embodiments, the conductive paths of the reinforcement element form terminals of the monitor interface. In embodiments, the reinforcement element is a flex circuit.

In embodiments, the wound dressing (or sensor pack layered with the wound dressing) of the wound dressing system comprises a monitor interface. In embodiments, the monitor interface is configured for electrically and/or mechanically connecting the wound dressing (at least the electrode assemblies) to a monitor device. In embodiments, the monitor interface is configured for wirelessly connecting the wound dressing/sensor pack to the monitor device. Thus, the monitor interface of the wound dressing is configured to electrically and/or mechanically couple the wound dressing/sensor pack and the monitor device.

In embodiments, the monitor interface of the wound dressing/sensor pack comprises, e.g. as part of a first connector of the monitor interface, a coupling part for forming or configured for forming a mechanical connection, such as a releasable coupling between the monitor device and the wound dressing. The coupling part can be configured to engage with a coupling part of the monitor device for releasably coupling the monitor device to the wound dressing. In embodiments, the coupling part is attached, such as glued, welded, press-fitted, or soldered, to the proximal and distal electrode assembly. In one or more exemplary wound dressings, the coupling part extends through the top layer opening, i.e. a part of the coupling part can extend on the proximal side of the top layer and a part of the coupling part can extend on the distal side of the top layer. The coupling part can be attached such as glued, welded, press-fitted, or soldered, to the top layer. In one or more exemplary wound dressings, the first part of the electrode assembly extends into the coupling part, i.e. the coupling part can accommodate at least a part of the first part of the electrode assembly.

In embodiments, the monitor interface of the wound dressing/sensor pack comprises, e.g. as part of a first connector of the monitor interface, a plurality of terminals, such as two, three, four, five, six, seven, eight or more terminals, for forming or configured to form electrical connections with respective terminals of the monitor device. In embodiments, the monitor interface comprises a ground terminal element forming a ground terminal. In embodiments, the monitor interface comprises a first terminal element forming a first terminal, a second terminal element forming a second terminal and optionally a third terminal element forming a third terminal. The monitor interface can comprise a fourth terminal element forming a fourth terminal and/or a fifth terminal element forming a fifth terminal. The monitor interface optionally comprises a sixth terminal element forming a sixth terminal. In embodiments, the terminal elements of the monitor interface contacts respective electrodes of the wound dressing, i.e. the proximal electrode assembly and/or the distal electrode assembly. In embodiments, a first intermediate element is arranged between the terminal elements and the first adhesive layer. In embodiments, the first intermediate element covers or overlaps terminal element(s) of the wound dressing when seen in the axial direction. Thus, the first adhesive layer can be protected or experience more evenly distributed mechanical stress from the terminal elements of the wound dressing, in turn reducing the risk of terminal elements penetrating or otherwise damaging the first adhesive layer. The first intermediate element can protect or mechanically and/or electrically shield the first adhesive layer from the terminal elements of the wound dressing.

In embodiments, connection parts of electrodes of the proximal and/or distal electrode assembly form respective terminals of the monitor interface. In embodiments, the coupling part(s), such as the monitor device coupling part and/or the coupling part of the wound dressing, forms a USB type plug or port. For example, the coupling part(s) can conform with a USB standard.

In embodiments, the monitor device of the wound dressing system comprises a processor, a memory, and a first interface. In embodiments, the components of the monitor device are contained in a housing, i.e. the monitor device comprises a housing. In embodiments, the monitor device comprises two or more interfaces. In embodiments, the memory is configured to store wound data and/or parameter data based on the wound data. In embodiments, the first interface is connected to the processor and the memory. In embodiments, the first interface is configured for collecting sensor data from the wound dressing (the electrode assemblies of the wound dressing) coupled to the first interface. Thus, in embodiments, the proximal and/or the distal electrode assembly are coupled to the first interface, e.g. through an electrical connection through the monitor interface as disclosed above.

In embodiments, sensor data comprises data from/pertaining to the proximal and/or distal electrode assemblies of the wound dressing. In embodiments, the sensor data comprises first sensor data from/pertaining to the proximal electrode assembly and second sensor data from/pertaining to the distal electrode assembly. In embodiments, sensor data comprises information pertaining to a resistance across/between two or more electrodes within an electrode assembly (the proximal or distal electrode assembly). In embodiments, resistance across/between two or more electrodes depends on the surroundings, e.g. presence of exudate from the wound (exudate uptake in the wound dressing) and hence, the sensor data comprises information pertaining to the presence and/or amount of exudate present in the vicinity of a certain set of electrodes, e.g. adjacent electrodes within an electrode assembly or across electrode assemblies.

In embodiments, exudate from the wound can form a (liquid) bridge/path/pathway from a primary electrode to a secondary electrode of the proximal or the distal electrode assembly (i.e. of the first plurality of electrode or the second plurality of electrodes), the liquid bridge thereby short-circuiting the primary electrode and the secondary electrode when the monitor device is applying a potential difference across the plurality of electrodes. In the following, the occurrence of a short-circuit is referred to as a short-circuiting event and hence refers to a situation where current passes through a path of low resistance (e.g. through exudate absorbed by the wound dressing, i.e. exudate uptake) rather than through the powered circuit. In particular, a short-circuit as described above is referred to as a short-circuiting event "across" a primary and a secondary electrode, meaning that a short-circuit is established between the primary electrode and the secondary electrode. In embodiments, the primary and secondary electrodes of the first or second plurality of electrode are specific electrodes of the first or second plurality of electrodes, such that they are identifiable and distinguishable by the monitor device. Thus, in embodiments, the monitor device is preprogrammed with the specific layout of electrodes of the first and second plurality of electrodes, such that the monitor device is capable of identifying which electrodes are short-circuiting/- circuited and where these electrodes are arranged relative to a specific wound dressing. Thereby, the monitor device is capable - through a configuration and method as described in the following - of mapping the short-circuiting events and determine where exudate may be present. For example, in a situation where the first (or second) plurality of electrodes comprises a first, a second, a third, a fourth, and a fifth electrode, the primary electrode can be any of the first, the second, the third, the fourth, and the fifth electrode, and the secondary electrode can be any of the first, the second, the third, the fourth, and the fifth electrode, except for the electrode denoted the primary electrode. In most situations, the primary and secondary short-circuited electrode are adjacent, since this constitutes the most common situation; exudate forming a bridge between adjacent electrodes within an electrode assembly.

In embodiments, information on a short-circuiting event within the proximal electrode assembly is included in first sensor data. In embodiments, by including is meant that data/information pertaining to the short-circuiting event is stored/recorded in the memory of the monitor device. In embodiments, data/information pertaining to a short-circuiting event as included in the first sensor data includes identification of the short-circuited electrodes and the time on which the short-circuiting event occurred. In embodiments, information on a short-circuiting event within the distal electrode assembly is included in second sensor data. In embodiments, data/information pertaining to a short-circuiting event included in the second sensor data includes identification of the short-circuited electrodes and the time on which the short-circuiting event occurred. In embodiments, information on a short-circuiting event between the proximal electrode assembly and the distal electrode assembly is included in a common sensor data set. In embodiments, data/information pertaining to a short-circuiting event included in the common sensor data set includes identification of the short-circuited electrodes of the proximal and distal electrode assembly and the time on which the short-circuiting event occurred.

In embodiments, the monitor device is configured to detect inter-electrode assembly short-circuiting events, i.e. short-circuiting events involving at least a primary electrode of the second plurality of electrodes and a primary electrode of the second plurality of electrodes. Such an inter-electrode assembly short-circuiting event can be the result of exudate forming a liquid path from at least one electrode of the first plurality of electrodes to at least one electrode of the second plurality of electrodes. In embodiments, such inter-electrode assembly short-circuiting events can narrow down the first region further, as such first region can now be reduced to the region where a single primary electrode of the proximal electrode assembly is short-circuited with a single primary electrode of the distal electrode assembly. Thus, the exudate uptake pattern can be narrowed down to the first region where the primary electrode of the proximal electrode assembly intersects the primary electrode of the distal electrode assembly. The inter-electrode assembly short-circuiting event forms a joint electrode comprising the involved electrodes. In embodiments, the monitor device is configured to differentiate short-circuiting events and to compare the short-circuiting electrodes both within an electrode assembly (the proximal or distal) and across electrode assemblies (the proximal and distal) in order to establish a first region and to indicate an exudate uptake pattern based on such first region. The following discussion mainly relates to determining a first region based on a short-circuiting event within the first plurality of electrodes and on a short-circuiting event within the second plurality of electrodes, but it should be noted that the system may as well be configured to determine a first region based on an inter-electrode assembly short-circuiting event, i.e. the system may be configured to determine a first region based on a short-circuiting event across a primary electrode of the first plurality of electrodes and a primary electrode of the second plurality of electrodes. In such configuration, the first region is at least the point where such primary electrodes of the first and second plurality of electrodes cross/intersect when seen in the direction normal to the appliance plane.

In embodiments, the monitor device is configured to detect and include in the first sensor data a short-circuiting event across a primary and a secondary electrode of the first plurality of electrodes and to detect and to include in the second sensor data a short-circuiting event across a primary and secondary electrode of the second plurality of electrodes. In embodiments, detecting a short-circuiting event comprises, by means of a power source included in the monitor device, applying a potential difference across the electrodes of the first plurality of electrodes and the second plurality of electrodes, monitoring the potential difference, and detect the occurrence of anomalies, such as a short-circuiting event. Thereby, the monitor device is capable of sensing/detecting a short-circuiting event and the involved electrodes.

The monitor device is configured to determine a first region of the wound dressing, wherein the first region is a region where the short-circuited primary and secondary electrodes of the first plurality of electrodes and the short-circuited primary and secondary electrodes of the second plurality of electrodes overlap in the normal direction (i.e., in the direction normal to the appliance plane). Thus, the first region is a region of the wound dressing both comprising short-circuited electrodes of the first plurality of electrodes (proximal electrode assembly) and short-circuited electrodes of the second plurality of electrodes (distal electrode assembly). As previously disclosed, the proximal and distal electrode assemblies, and hence the first and secondary plurality of electrodes, are layered and spaced apart by a first material in a direction normal to the appliance plane. Thus, once a short-circuiting event has been detected in both of the assemblies (hence, each of the first and second plurality of electrodes), the monitor device is configured to compare such short-circuiting events in order to determine a first region of the wound dressing, in particular of the electrode assemblies, where an overlap between the short-circuited electrodes exist. In other words, the monitor device is configured to map the short-circuited electrodes of the distal electrode assembly onto the short-circuited electrodes of the proximal electrode assembly, such that the position of the cause of the short-circuits (most likely; an exudate uptake in the wound dressing, e.g. exudate absorbed by the absorbent core layer) can be narrowed down to the first region as defined. Thereby, each of the electrode assemblies can be provided in a simple array, e.g. as parallel electrodes, having a certain resolution in terms of their ability to pin-point an exudate uptake pattern, but combined, the resolution can be improved.

The monitor device is configured to indicate an exudate uptake pattern of the wound dressing based on at least the first region, i.e. the first region as defined above. In embodiments, by indicating an exudate uptake pattern is meant to communicate and/or visualize the exudate uptake pattern, e.g. in a (graphical) user interface. In embodiments, by indicating an exudate uptake pattern is meant to transmit a signal indicative of the exudate uptake pattern from the monitor device to an accessory device and to visualize the exudate uptake pattern on a user interface of said accessory device. In embodiments, by indicating an exudate uptake pattern is meant to transmit information pertaining to an exudate uptake pattern to an accessory device and optionally to visualize the information in a (graphical) user interface thereof. Thus, by the monitor device indicating an exudate uptake pattern does not necessarily comprise the monitor device visualizing the exudate uptake pattern.

In embodiments, the monitor device is configured to predict and/or indicate an exudate uptake pattern based on historic data recorded through the proximal and/or distal electrode assemblies. In embodiments, historic data includes data pertaining to short-circuiting events within the electrode assemblies.

In embodiments, indicating an exudate uptake pattern comprises indicating, e.g. visualizing, the first region of the wound dressing in a graphical representation of said wound dressing. In embodiments, the graphical representation is provided in a graphical user interface of an accessory device, such as in an app of a smartphone/-watch.

In embodiments, the monitor device is configured, e.g. by means of the processor, to compare the first sensor data and the second sensor data and to establish a first region where the (short-circuited) primary and secondary electrodes of the first plurality of electrodes and the (short-circuited) second plurality of electrodes overlap. In embodiments, the monitor device is capable of storing first sensor data (comprising information pertaining to a short-circuiting event across a primary and a secondary electrode of the first plurality of electrodes) in the memory and obtain/load such first sensor data once the monitor device detects a short-circuiting event across a primary and a secondary electrode of the second plurality of electrodes. Thus, once the monitor device has detected a short-circuiting event in both the proximal electrode assembly and the distal electrode assembly, it is configured to compare the respective (first and second) sensor data. In embodiments, such comparison includes determining which electrodes of the electrode assemblies are short-circuited and identifying a first region of the wound dressing wherein the short-circuited electrodes of the proximal electrode assembly and the distal electrode assembly are overlapping in the normal direction, i.e., when viewing the wound dressing in a direction normal to the appliance plane. In other words, in embodiments, since the proximal and distal electrode assemblies are layered (and spaced apart by a first material/gap in the normal direction) as previously described, and since the first and second plurality of electrodes are, in embodiments, rotated (previously described), it is possible to establish such overlapping region. Based on at least the first region, the monitor device is configured to determine a (probable) exudate uptake pattern of the wound dressing, such as of the absorbent core layer of the wound dressing. In embodiments, by overlapping is meant that short-circuited electrodes of the first plurality of electrodes and short-circuited electrodes of the second plurality of electrodes intersect in a certain (first) region when seen in a direction being normal/perpendicular to the appliance plane and hence the planes spanned by the electrode assemblies. In embodiments, such certain region is referred to as the first region, and hence pertains to a region - location - in a two-dimensional representation of the wound dressing, where a primary and secondary electrode of the first plurality of electrodes and a primary and secondary electrode of the second plurality of electrodes are all short-circuited.

Due to the arrangement of the first plurality of electrodes and the second plurality of electrodes, a short-circuiting event within, for example, the proximal electrode assembly, reveals a certain amount of information pertaining to the location of the wetting/presence of exudate (a certain resolution) relative to the wound dressing and wound as such. In embodiments, such certain information is limited to identifying that exudate uptake or wetting has occurred somewhere along the primary and secondary electrode (which can extend from a first edge to an opposite second edge of the wound dressing/sensor pack aligned with wound dressing), but not necessarily at what point along said electrodes. However, by introducing a distal electrode assembly comprising a second plurality of electrodes rotated relative to the first plurality of electrodes as previously disclosed, it is possible to deduce the location of the exudate uptake pattern/wetting pattern to a greater detail (improved resolution). Thus, through the knowledge (e.g. as contained in the first sensor data according to embodiments) of a short-circuiting event across a primary electrode and a secondary electrode in the proximal electrode assembly and the knowledge (e.g. as contained in the second sensor data according to embodiments) of a short-circuiting event across a primary electrode and a secondary electrode in the distal electrode assembly, it is possible to determine a (probable) exudate uptake pattern of the wound dressing, such as of the absorbent core layer. The exudate uptake pattern is based on at least the first region. In embodiments, the exudate uptake pattern is based on the first region, or on the first region and the first sensor data, or on the first region and the second sensor data, or on the first region, on the first sensor data, and on the second sensor data. By probable is meant that the actual exudate uptake pattern can be different if conditions change within the wound and/or wound dressing, such as in a time-delay from when the short-circuiting event occurred in the proximal electrode assembly to when the short-circuiting event occurred in the distal electrode assembly. However, in embodiments, it is possible for the monitor device to determine a probable exudate uptake pattern by taking into account the data contained in the sensor data. In embodiments, the monitor device is configured to indicate the exudate uptake pattern and a certain uncertainty of the exudate uptake pattern. In embodiments, the certain uncertainty is based on the sensor data and/or a time-delay.

In embodiments, the monitor device is configured to determine the exudate uptake pattern of the wound dressing through the processor applying a processing scheme to the sensor data. In embodiments, the processing scheme is an algorithm configured to compare the first and second data and determine the exudate uptake pattern.

In embodiments, by an exudate uptake pattern is meant the location of the exudate uptake/wetting relative to a physical shape/extension of the wound dressing. In embodiments, by an exudate uptake pattern is meant the location of the exudate uptake/wetting relative to a physical shape of the wound dressing and the shape of the wetting. Thereby, the user and/or a health care professional is capable of telling where (i.e., the location relative to the shape/boundaries/edges of the wound dressing or relative to certain characteristics of the wound itself) an exudate uptake/wetting occurs without removing the wound dressing, and in addition; is capable of identifying the extent of exudate uptake from the extent/shape. In embodiments, the location and/or extent/shape of an exudate uptake of a wound dressing reveals information on the condition of the wound as such, e.g. a healing progression.

In an embodiment, the monitor device is further configured to assign a first timestamp to the short-circuiting event across a primary electrode and a secondary electrode of the first plurality of electrodes and to assign a second timestamp to the short-circuiting event across a primary electrode and a secondary electrode of the second plurality of electrodes, and to determine a first time-delay between said short-circuiting events based on said assigned first and second timestamps.

In embodiments, the first and second timestamps are recorded in a memory of the monitor device. In embodiments, a processor of the monitor device is configured to load the timestamps and to calculate the first time-delay. In embodiments, the first time-delay is calculated by subtracting the first timestamp from the second timestamp. Thus, in embodiments, the processor is capable of determining the first time-delay upon detection of a short-circuiting event within the proximal electrode assembly (e.g., the first timestamp is included in first sensor data) and the distal electrode assembly (e.g., the second timestamp is included in second sensor data). In embodiments, the first time-delay forms basis for further analysis.

In embodiments, the time-delay is indicative of the rate of exudate production. In embodiments, the rate of exudate production is derived from at least the first time-delay, e.g. by means of a processor of the monitor device. In embodiments, the rate of exudate production is derived from at least the first time-delay, the width/size of the first gap, and material properties of the first material occupying the first gap, e.g., the absorbent core layer or a spacer element.

In an embodiment, the monitor device is configured to indicate the exudate uptake pattern of the wound dressing based on at least the (determined) first region and the (determined) first time-delay.

In embodiments, the first time-delay as introduced above constitutes an additional parameter forming basis for the indicated exudate uptake pattern. For example, if the exudate uptake/wetting of the wound dressing, e.g. of the absorbing core layer, is slow, e.g. if the wound does not produce a large amount of exudate, a relatively long time can pass (large time-delay) between a short-circuiting event is detected within the proximal electrode assembly and a short-circuiting event is detected within the distal electrode assembly. Hence, in the meantime, conditions of the wound can change, thereby reducing the probability of the exudate uptake pattern being within the first region. Hence, by including the time delay as a parameter in determining the exudate uptake pattern, it is possible to communicate to the user and/or health care professional that the exudate uptake pattern is more diffuse than what would otherwise be communicated from only considering the first region. On the contrary, a small time-delay can be indicative of a large amount of exudate being expelled from the wound, hence prompt a warning that the wound dressing should be changed soon, or sooner than otherwise expected. In embodiments, the time-delay is dependent on the width/size of the first gap and/or the material separating the proximal electrode assembly and the distal electrode assembly.

In an embodiment, the electrodes of the first plurality of electrodes are mutually parallel and extending in a first direction, the electrodes of the second plurality of electrodes are mutually parallel and extending in a second direction, and the first direction and the second direction are non-parallel. Thereby, a matrix configuration of overlapping electrodes is formed. The overlap refers to the situation wherein the electrode assemblies/the wound dressing are viewed in a direction being normal/perpendicular to the appliance plane of the wound dressing (or of the sensor pack aligned with wound dressing) and hence of the geometric planes spanned by the (layered) first and second plurality of electrodes. In embodiments, the first and the second direction are orthogonal.

In embodiments, by non-parallel is meant that an angle between the first direction and the second direction is selected in the open interval (0°,180°), also denoted ]0°,180°[. By orthogonal is meant that an angle between the first and the second direction is 90°.

By the electrodes being mutually parallel is meant that the electrodes are separated by a constant first distance, such as by 1 mm, or by 2 mm, or by 3 mm, or by 4 mm, or by 5 mm, or by 6 mm, or by 7 mm, or by 8 mm, or by 9 mm, or by 10 mm, or by a distance selected between 1 mm and 20 mm, such as between 10 mm and 20 mm. In embodiments, the electrodes are separated by a varying distance. In embodiments, by providing a varying distance, the distance between the electrodes in a centre region of the electrode assembly can be smaller than the distance between electrodes in a boundary region. In embodiments, the opposite situation can be the case, i.e. where the distance between electrodes in a boundary region is smaller than the distance between electrodes in a centre region. By providing a varying distance between the parallel electrodes, a certain sensitivity can be achieved in a certain region(s) of the wound dressing without increasing the number of electrodes, as less electrodes thereby can be provided in other regions.

In embodiments, the first direction is orthogonal/perpendicular to the second direction when seen in a direction normal/perpendicular to the planes spanned by the electrode assemblies. In other words, the electrodes of the second plurality of electrodes extend in a direction perpendicular to the direction in which the electrodes of the first plurality of electrodes extend, i.e., the appliance plane. In further other words, when viewed in a direction normal to the geometrical planes spanned by the first and second plurality of electrodes, the first and second plurality of electrodes are perpendicular. Thereby, the electrodes form an array comprising quadrilateral or square fields bound by electrodes.

Thereby, when comparing the first and second sensor data comprising information pertaining to short-circuiting events, the monitor device is capable of narrowing down the region of exudate uptake to a relatively small region: namely the first region wherein electrodes of the first plurality of electrodes and electrodes of the second plurality of electrodes are both short-circuited - this first region thereby representing the most probable exudate uptake pattern.

In embodiments, the first direction is rotated relative to the second direction by an angle selected between 0 degrees and 90 degrees, such as between 30 degrees and 60 degrees. Thereby, a matrix comprising diamond shaped (i.e., in the shape of a rhombus) fields bound by electrodes is formed. Such configuration may be beneficial in certain shapes of the wound dressing as such.

In an embodiment, the first material is at least a portion of the absorbent core layer. In other words, in embodiments, the first gap between the proximal electrode assembly and the distal electrode assembly is occupied by at least a portion of the absorbent core layer. In embodiments, the first material is the (entirety of the) absorbent core layer.

As previously disclosed, the proximal and distal electrode assemblies are layered and spaced apart by a first material, thereby forming a first gap between the electrode assemblies. According to this embodiment, the proximal electrode assembly and the distal electrode assembly are spaced apart by at least a portion of the absorbent core layer, such as the entire absorbent core layer. In embodiments, the (entire) absorbent core layer occupies the first gap formed between the layered electrode assemblies. In embodiments, the proximal electrode assembly and/or the distal electrode assembly is embedded within the absorbent core layer, such that both sides of the electrode assemblies are adjacent a part of the absorbent core layer, meaning that only a portion of the absorbent core layer spaces the proximal electrode assembly from the distal electrode assembly.

In embodiments, the proximal electrode assembly is arranged on the proximal side, such on as the proximal surface, of the absorbent core layer, and the distal electrode assembly is arranged on the distal side, such as on the distal surface, of the absorbent core layer. Thereby, the entire absorbent core layer is contained/sandwiched between the proximal electrode assembly and the distal electrode assembly. In embodiments, the distal electrode assembly is arranged on a proximal side of a top layer and on a distal side of the absorbent core layer.

Thereby, exudate can initially short-circuit two or more electrodes of the proximal electrode assembly (closest to the wound relative to the distal electrode assembly when the wound dressing is applied to a wound of a user), followed by (when the absorbent core layer has absorbed a sufficient amount of exudate) short-circuiting two or more electrodes of the distal electrode assembly. A time delay, as previously described, can form due to the time it takes the absorbent core layer to absorb a sufficient amount of exudate necessary to generate a short-circuiting event across a primary and a secondary electrode of the distal electrode assembly.

Thereby is provided a way of introducing a time delay between short-circuiting events in the proximal electrode assembly and the distal electrode assembly, the time delay, in embodiments, being a parameter when determining an exudate uptake pattern of the wound dressing or a rate of exudate production. Thereby is provided a wound dressing facilitating determination of an exudate uptake pattern of the absorbent core layer and of a rate of exudate production.

In an embodiment, the first material is a spacer element. In other words, in embodiments, the first gap between the proximal electrode assembly and the distal electrode assembly is occupied by a spacer element.

In embodiments, the proximal electrode assembly is arranged on a proximal surface of the spacer element and the distal electrode assembly is arranged on a distal surface of the spacer element. Thus, in embodiments, a thickness of the spacer element equals the width/size of the first gap.

In embodiments, the spacer element is part of a sensor pack configured to be layered/aligned with an absorbent core layer of a wound dressing.

In embodiments, the spacer element is a component of the wound dressing, e.g. as a layered component. In embodiments, the spacer element is the support layer for the distal and/or proximal electrode assembly. In embodiments, the spacer element comprises a polymeric film, such as a (thermoplastic) polyurethane film. In embodiments, adhesive is provided on the proximal surface of the spacer element and/or on the distal surface of the spacer element. In embodiments, the spacer element is arranged on a proximal side of the absorbent core layer. In embodiments, the spacer element is arranged on a distal side of the absorbent core layer. In embodiments, the spacer element is less absorbing than the absorbent core layer. In embodiments, the spacer element is insulative and non-absorbing. In embodiments, the spacer element serves to space the proximal electrode assembly from the distal electrode assembly, such as to avoid short-circuiting between electrodes of the first plurality of electrodes and electrodes of the second plurality of electrodes. In embodiments, the spacer element comprises perforations, thereby allowing exudate to pass through, such as to allow exudate to reach the distal electrode assembly, and/or, in situations where the spacer element is arranged on a proximal side of the absorbent core layer, to reach the absorbent core layer. In embodiments, the perforations are offset from any electrodes of the first plurality of electrodes and the second plurality of electrodes. For example, the perforations are arranged within the fields bound by the perpendicular/rotated electrodes as described above. For example, the perforations are offset from where the electrodes cross each other. Thereby, the risk of unintentional short-circuits forming across an electrode within the first plurality of electrodes and an electrode within the second plurality of electrodes is reduced. Further, by spacing the proximal electrode assembly from the distal electrode assembly by means of a (insulative and non-absorbing) spacer element, a time delay from a short-circuiting event across electrodes of the first plurality of electrodes and electrodes of the second plurality of electrodes can be reduced, compared to a situation where exudate has to be absorbed by the absorbent core layer before reaching the distal electrode assembly. Thereby, a rate of exudate production can be derived more accurately.

In an embodiment, the monitor device is further configured to detect one or more additional short-circuiting events across two or more electrodes of the first and/or second plurality of electrodes.

Thus, the monitor device is configured to detect a second short-circuiting event across two or more electrodes of the first plurality of electrodes and/or across two or more electrodes of the second plurality of electrodes. In embodiments, a second short-circuiting event across electrodes within the first (or second) plurality of electrodes is between the primary/secondary electrode (now effectively a joint electrode due to being short-circuited prior to the second short-circuiting event in the same electrode assembly) and a tertiary electrode of the first (or second) plurality of electrodes, or between the primary/secondary electrode (now a joint electrode due to being short-circuited) and a quaternary electrode, and so forth. In embodiments, the second short-circuiting event across electrodes within the first (or second) plurality of electrodes is across one of the already short-circuited electrodes (primary and secondary electrode) and a third electrode, or the second short-circuiting event across electrodes within the first (or second) plurality of electrodes is across new electrodes, not previously short-circuited (i.e., excluding the primary and secondary electrodes).

In embodiments, a second short-circuiting event within the first plurality of electrodes can occur/be detected before a short-circuiting event has occurred/been detected within the second plurality of electrodes without affecting the functionality of the monitor device. Rather, in embodiments, since the monitor device is configured to determine a first region where the short-circuited electrodes of the electrode assemblies overlap, additional short-circuiting events within the first/second plurality of electrodes merely contributes to (and, potentially, serves to improve, such as to make it more accurate) the indication of the exudate uptake pattern.

Thereby is provided means for continuously monitoring the wound dressing, such as to monitor changes or propagation in the exudate uptake and/or production.

In an embodiment, the monitor device is further configured to indicate a propagation of the exudate uptake pattern of the wound dressing based on the detection of one or more additional short-circuiting events across two or more electrodes of the first and/or second plurality of electrodes.

Additional short-circuiting events can be the result of a propagation of the exudate uptake, e.g. a spreading due to large amounts of exudate being exuded from the wound or due to changing conditions of the wound as such. By continuously monitoring the wound dressing (i.e., detecting short-circuiting events), the monitor device is capable of detecting such additional short-circuiting events and determine a (new/updated) first region, wherein all the short-circuited electrodes of the first plurality of electrodes overlap (in the normal direction) with all the short-circuited electrodes of the second plurality of electrodes, and, based on the new/updated first region, indicate a (new/updated) exudate uptake pattern.

Further described herein is a sensor pack for a wound dressing of a wound dressing system is disclosed. The sensor pack comprises a spacer element having a proximal surface and distal surface, a proximal electrode assembly comprising a first plurality of electrodes, and a distal electrode assembly comprising a second plurality of electrodes. The proximal electrode assembly is arranged on the proximal surface of the spacer element and the distal electrode assembly is arranged on the distal surface of the spacer element.

Thereby is provided a separate item/appliance suitable for implementation/use in a (conventional) wound dressing of a wound dressing system according to the invention. Thus, it is appreciated that features and embodiments disclosed in relation to the wound dressing system according to the invention are also applicable to the above described sensor pack for a wound dressing.

By a sensor pack is meant a separate item, in particular a substantially flat/planar item/sensing layer suitable for being layered with a conventional wound dressing, e.g. by arranging/aligning the sensor pack on a distal or proximal side of an absorbent core layer of a conventional wound dressing. Thus, the sensor pack is a flexible item/layer facilitating detection of short-circuiting events across the implemented electrodes. The sensor pack is configured to be arranged/layered with a wound dressing, such as configured to be arranged on a distal side of an absorbent core layer of a wound dressing. Thus, the sensor pack can be considered a sub-item of the previously disclosed wound dressing of the wound dressing system, the sensor pack providing for at least a more efficient production process. Thereby, a conventional wound dressing can be equipped with the sensing capabilities as described in relation to the invention. In other words, the sensor pack comprises the electrodes and the first gap (as formed by means of the spacer element of the sensor pack) of the wound dressing system according to the first aspect to work, whereas a conventional wound dressing comprises the absorbent core layer, and potentially additional features according to embodiments, such as a backing layer/top film and/or an adhesive. Thus, a sensor pack and a wound dressing in combination is suitable for use in the wound dressing system according to the invention: the sensor pack provides/carries the electrode assemblies for the wound dressing. During manufacturing, the sensor pack can be arranged on a side, e.g. the distal side, of the absorbent core layer of a conventional wound dressing, whereby said wound dressing becomes suitable for use in a wound dressing system according to the invention. Thus, the sensor pack facilitates an easy production process, since the sensor pack can be assembled separate from the manufacturing of the wound dressing, and subsequently be incorporated, e.g. in a single step of the production process, into the wound dressing. In embodiments, the sensor pack is non-absorbent. In embodiments, the sensor pack does not comprise an absorbent core layer. In embodiments, the sensor pack in non-adhesive.

The spacer element of the sensor pack provides the aforementioned first gap in a direction normal to the appliance plane of the wound dressing (when the sensor pack is layered with such a wound dressing). Thus, the sensing pack and an absorbent core layer may, in combination, provide a wound dressing having sensing capabilities according to the invention.

In an embodiment, the electrodes of the first plurality of electrodes are mutually parallel and extending in a first direction, the electrodes of the second plurality of electrodes are mutually parallel and extending in a second direction, and the first direction and the second direction are non-parallel.

Thereby, a matrix configuration of overlapping electrodes is formed, as discussed above for the invention. The overlap refers to the situation wherein the electrode assemblies/the sensor pack (or wound dressing when the sensor pack is incorporated into/layered with said wound dressing) are viewed in a direction being normal/perpendicular to appliance plane of the wound dressing/sensor pack and hence of the geometric planes spanned by the (layered) first and second plurality of electrodes. In embodiments, the first and the second directions are orthogonal.

In embodiments, by non-parallel is meant that an angle between the first direction and the second direction is selected in the open interval (0°,180°), also denoted ]0°,180°[. By orthogonal is meant that an angle between the first and the second direction is 90°.

In an embodiment, the spacer element comprises a polymeric film material. In embodiments, the spacer element is a polymeric film.

In embodiments, the spacer element comprises a polymeric film, such as a (thermoplastic) polyurethane film. In embodiments, the first plurality of electrodes is arranged directly on, such as printed on, the proximal surface of the spacer element. In embodiments, the second plurality of electrodes is arranged directly on, such as printed on, the distal surface of the spacer element. Thus, the spacer element can be considered a support layer of the distal and/or proximal electrode assembly. In embodiments, the spacer element is a laminate of at least two polymeric films. In embodiments, the thickness of the spacer element is less than 100 µm, such as less than 50 µm, such 30 µm, thereby providing for a particularly flexible sensor pack.

Also disclosed herein is a method for determining an exudate uptake pattern of a wound dressing is disclosed. The wound dressing extends in an appliance plane and comprises an absorbent core layer having a proximal side and a distal side, a proximal electrode assembly comprising a first plurality of electrodes, and a distal electrode assembly comprising a second plurality of electrodes. The proximal electrode assembly and the distal electrode assembly are layered and spaced apart by a first material in a direction normal to the appliance plane. In embodiments, the electrode assemblies are provided in a sensor pack layered with the wound dressing comprising the absorbent core layer. Thus, the method incorporates the features of a wound dressing, in particular of a wound dressing for a wound dressing system, as previously disclosed in relation to the invention. The method is performed in a monitor device connected to the wound dressing or sensor pack layered with the wound dressing. In embodiments, the method is performed in a monitor device comprising a processor, a memory, and a first interface connected to the processor and the memory. The first interface is configured for collecting sensor data from the proximal electrode assembly and the distal electrode assembly of the wound dressing or sensor pack, when the wound dressing/sensor pack is coupled to the first interface. The sensor data comprises first sensor data from the proximal electrode assembly and second sensor data from the distal electrode assembly.

The method, performed in a monitor device connected to the wound dressing, comprises the steps of detecting a short-circuiting event across a primary and a secondary electrode of the first plurality of electrodes, detecting a short-circuiting event across a primary and a secondary electrode of the second plurality of electrodes, determining a first region of the wound dressing (or sensor pack layered with the wound dressing), wherein the first region is a region where the primary and secondary electrodes of the first plurality of electrodes and the primary and secondary electrodes of the second plurality of electrodes overlap in the direction normal to the appliance plane, and indicating an exudate uptake pattern of the wound dressing based on at least the first region.

Thereby is provided a method for determining an exudate uptake pattern of a wound dressing utilizing a wound dressing system according to the invention. Thus, it is appreciated that the wound dressing system, the wound dressing or sensor pack and wound dressing in combination, and the monitor device, including features and embodiments thereof, as disclosed according to the , invention are applicable to the methoc as disclosed.

In embodiments, by indicating an exudate uptake pattern is meant to visualize the exudate uptake pattern in a (graphical) user interface. In embodiments, by indicating an exudate uptake pattern is meant to transmit a signal from the monitor device to an accessory device and to visualize the exudate uptake pattern on a (graphical) user interface of said accessory device. In embodiments, by indicating an exudate uptake pattern is meant to transmit information indicative of an exudate uptake pattern to an accessory device and optionally to visualize the information in a (graphical) user interface thereof. Thus, by the monitor device indicating an exudate uptake pattern does not necessarily comprise the monitor device visualizing the exudate uptake pattern. In embodiments, by indicating is meant to make the user and/or health care professional aware of an exudate uptake pattern, including a mere existence of an exudate uptake pattern, e.g. through an audio signal and/or haptic feedback.

In embodiments, indicating an exudate uptake pattern comprises indicating, e.g. visualizing, the first region of the wound dressing in a graphical representation of said wound dressing. In embodiments, the graphical representation is provided in a graphical user interface of an accessory device, such as in an app of a smartphone/-watch.

In alternative embodiments, the method is performed in an accessory device, such as a handheld device, such as a smartphone comprising means for carrying out the method, e.g., through a processor and a memory. In such embodiments, the monitor device comprises a wireless transmitter and is configured to relay (raw) sensor data to the accessory device.

In embodiments, the method includes the step of transmitting a representation of the exudate uptake pattern to an accessory device. In embodiments, the method includes the step of visualizing the representation of the exudate uptake pattern on a graphical user interface of the accessory device.

In embodiments, the method includes the step of determining an uncertainty of an indicated exudate uptake pattern.

In an embodiment, the method further comprises the step of assigning a first timestamp to the short-circuiting event across a primary electrode and a secondary electrode of the first plurality of electrodes, assigning a second timestamp to the short-circuiting event across a primary electrode and a secondary electrode of the second plurality of electrodes, and determining a first time-delay between said short-circuiting events based on said assigned first and second timestamps. In embodiments, the method comprises recording the first and second timestamps in a memory of the monitor device. In embodiments, the method comprises loading the timestamps and calculate the first time-delay, e.g. by means of a processor of the monitor device. In embodiments, the first time-delay is calculated by subtracting the first timestamp from the second timestamp. Thus, in embodiments, the processor is capable of determining the first time-delay upon detection of a short-circuiting event within the proximal electrode assembly (e.g., the first timestamp is included in first sensor data) and the distal electrode assembly (e.g., the second timestamp is included in second sensor data). In embodiments, the first time-delay forms basis for further analysis.

In embodiments, the method comprises deriving a rate of exudate production from the first time-delay. In embodiments, the rate of exudate production is derived from at least the first time-delay, the width/size of the first gap, and material properties of a material occupying the first gap, e.g. the absorbent core layer or a spacer element. In embodiments, the method comprises indicating the rate of exudate production, e.g. by transmitting a signal to an accessory device.

In an embodiment, the exudate uptake pattern of the wound dressing is based on at least the (determined) first region and the (determined) first time-delay. Thereby is provided a method for indicating an exudate uptake pattern of a wound dressing taking into account both the first region as previously described and the first time-delay. In embodiments, in cases where the first time-delay is large, the conditions of the wound is likely to have changed, thereby increasing an uncertainty of the determined first region. On the contrary, in embodiments, in cases where the first time-delay is small, the determined first region is likely to represent the actual condition of the wound. Thus, the first time-delay may be considered a parameter indicative of an (un)certainty of the determined first region. In embodiments, the monitor device is configured to indicate an uncertainty of the indicated exudate uptake pattern based on the first time-delay.

In an embodiment, the method comprises the additional step of detecting one or more additional short-circuiting events. Previous discussions on the detection of one or more additional short-circuiting events are considered applicable to this step of the method. Thereby is provided a method for continuously monitoring the wound dressing system and to base further/additional/future exudate uptake patterns on such one or more additional short-circuiting events.

In an embodiment, the method comprises the additional step of indicating a propagation of the exudate uptake pattern based on the one or more additional short-circuiting events. According to the embodiment, if one or more additional short-circuiting events are detected, the method is capable of determining a propagation of the exudate uptake pattern, i.e. to indicate a spreading of the exudate uptake pattern, e.g. caused by changing conditions of the wound as such. By detecting one or more additional short-circuiting events, it is, in embodiments, possible to determine a propagation of the exudate uptake pattern, as the one or more additional short-circuiting events are due to occur in a region outside the first region. Thus, a "new" first region can be considered the sum of the "old" first region and the region as defined by the one or more additional short-circuiting events. Hence, the "new" first region is larger than the "old" first region, and is indicative of a spreading of the exudate, e.g. in the absorbent core layer due to increasing absorption or increased/changed exudate production by the wound as such.

### Detailed description of the drawings

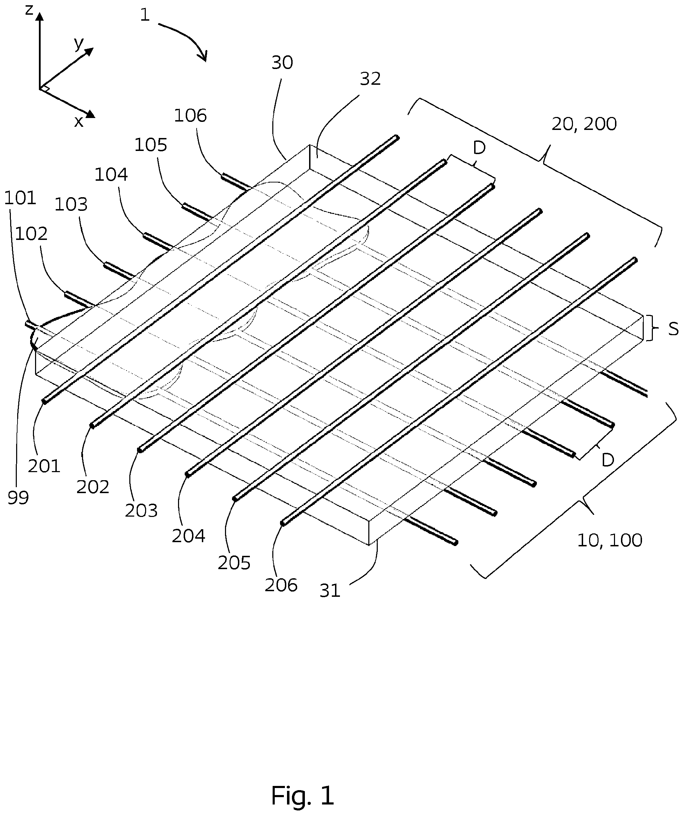

Fig. 1 illustrates a perspective view of a model of a wound dressing 1 for a wound dressing system according to an embodiment of the invention. The wound dressing 1 illustrates three components; a proximal electrode assembly 10, a distal electrode assembly 20, and an absorbent core layer 30 separating the proximal electrode assembly 10 from the distal electrode assembly 20. In embodiments, the absorbent core layer 30 is substituted with a spacer element, as will be covered by subsequent figures and discussions. Thus, in situations where the spacing feature/property of the absorbent core layer is considered, the discussion may as well apply to a spacer element. The absorbent core layer 30 comprises a proximal side 31 and a distal side 32. The proximal side 31 is configured to be oriented towards the skin (wound) surface of a user. In embodiments, a first adhesive layer is provided on the proximal side 31 of the absorbent core layer 30 for adhering the wound dressing 1 to the skin surface. In embodiments, a protective top film is provided on the distal side 32, the top film protecting the wound dressing 1 from external stress and/or dirt. In the embodiment, the proximal electrode assembly 10 is arranged on the proximal side 31 of the absorbent core layer 30, such as on a proximal surface of the absorbent core layer 30, and the distal electrode assembly 20 is arranged on the distal side 32 of the absorbent core layer 30, such as on the distal surface of the absorbent core layer 30. Thereby, the proximal electrode assembly 10 and the distal electrode assembly 20 are layered and separated/spaced apart by a first gap S; a thickness/width/size of which thereby equαlling a thickness of the absorbent core layer 30. Thus, in the embodiment, the absorbent core layer 30 occupies the first gap S. A coordinate system (x,y,z) is indicated in Fig. 1 to illustrate how the proximal 10 and distal electrode assembly 20 each span a geometrical plane, here both being parallel with the (x,y)-plane. The geometrical planes are mutually parallel and non-coinciding. The first gap S extends in a direction normal to a planar extension of the wound dressing 1 (also denoted the appliance plane of the wound dressing 1). Here, the appliance plane of the wound dressing 1 is the (x,y)-plane and hence, the normal direction is along (parallel to) the z-axis of the indicated coordinate system.

The proximal electrode assembly 10 comprises a first plurality of electrodes 100. The first plurality of electrodes 100 according to Fig. 1 comprises a first electrode 101, a second electrode 102, a third electrode 103, a fourth electrode 104, a fifth electrode 105, and a sixth electrode 106. The electrodes 101-106 of the first plurality of electrodes 100 are mutually parallel and separated by a first distance D.

The distal electrode assembly 20 comprises a second plurality of electrodes 200. The second plurality of electrodes 200 according to Fig. 1 comprises a first electrode 201, a second electrode 202, a third electrode 203, a fourth electrode 204, a fifth electrode 205, and a sixth electrode 206. The electrodes 201-206 of the second plurality of electrodes 200 are mutually parallel and likewise separated by a first distance D.

An amount of exudate 99 residing on the proximal side 31 of the absorbent core layer 30 and being in contact with the proximal electrode assembly 10 is illustrated. The exudate 99 may originate from an exuding wound adjacent to which the wound dressing 1 is arranged. Initially, the exudate 99 contacts a part of the electrodes 101-106 of the first plurality of electrodes 100. In particular, the exudate 99 forms a liquid bridge/path across all electrodes 101-106 of the first plurality of electrodes 100. When a voltage is applied the first plurality of electrodes 100 by means of a power source, e.g. a monitor device as will be described in greater detail in relation to subsequent figures, the presence of a liquid bridge will cause the electrodes 101-106 to form a short-circuit. A short-circuit is characterised by the current passing through a path of low resistance (e.g. through the exudate) rather than through the powered circuit. Thus, a short-circuit across the electrodes can be detected by means of an appropriate monitor device. The exudate 99 of Fig. 1 is shown to form a liquid bridge across all electrodes 101-106 of the first plurality of electrodes 100, thereby establishing a short-circuit across all electrodes 101-106. The detection (by a monitor device) of a short-circuit is denoted a short-circuiting event, and the monitor device can be configured to determine which electrodes are short-circuited. As shown in Fig. 1, the exudate 99 is only in contact with a certain portion of the electrodes 101-106 of the first plurality of electrodes 100. However, due to the separation/orientation and extent of the electrodes 101-106 (the same reasoning applies to the electrodes 201-206 of the second plurality of electrodes 200), a monitor device connected to the proximal electrode assembly 10 (and/or distal electrode assembly 20 when applying the same reasoning) would not immediately be able to tell where the short-circuiting event has occurred. Rather, the monitor device is able to detect which electrodes are short-circuited, but not where along their extent. Thus, in the situation shown in Fig. 1, a monitor device would assume - based on short-circuiting events detected from the proximal electrode assembly 10 only - that the entire proximal electrode assembly 10 is wetted by exudate 99, despite said exudate 99 only being present in a certain portion of the electrodes 101-106. According to embodiments of the invention, the distal electrode assembly 20 is layered with the proximal electrode assembly 10, rotated relative to the proximal electrode assembly 10, and spaced by a first gap S from said proximal electrode assembly 10. Thereby is provided a way to narrow down the possible region of exudate uptake/wetting. Namely, as shown in Fig. 1, the electrodes 201-206 of the second plurality of electrodes 200 belonging to the distal electrode assembly 20 are rotated relative to the electrodes 101-106 of the first plurality of electrodes 100. The electrodes 201-206 of the second plurality of electrodes 200 extend in a second direction, and the electrodes 101-106 of the first plurality of electrodes 100 extend in a first direction, the first and second directions being different. In Fig. 1, the second direction is orthogonal/perpendicular to the first direction when seen in the normal direction (i.e., parallel to the z-direction indicated by the coordinate system (x,y,z)). Due to this orientation of the first plurality of electrodes 100 relative to the second plurality of electrodes 200, a monitor device connected to both electrode assemblies 10,20 can be configured to narrow down the region of exudate uptake once a short-circuiting event is detected across two or more electrodes 201-206 of the second plurality of electrodes 200, i.e. when the exudate 99 has propagated through the absorbent core layer 30 and formed a liquid bridge between at least two electrodes of the second plurality of electrodes 200.

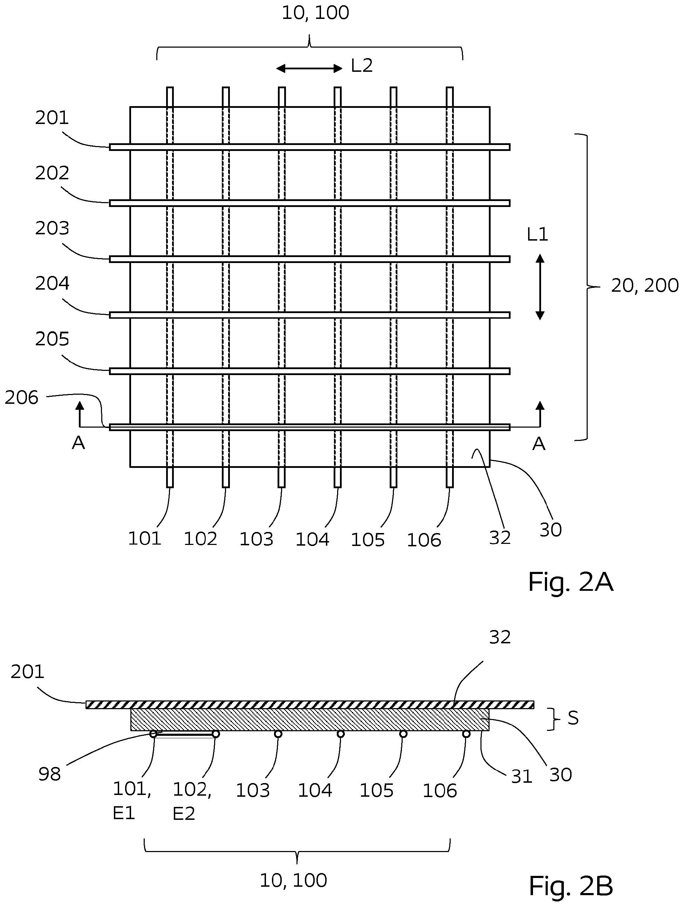

Fig. 2A illustrates a top view of the model of a wound dressing for a wound dressing system as shown in Fig. 1. Fig. 2B illustrates a cross-sectional view of the model of a wound dressing shown in Fig. 2A taken along the line A-A.

The top view of Fig. 2A highlights the distal electrode assembly 20 and the distal side 32 of the absorbent core layer 30. The proximal electrode assembly 10 is indicated by dashed lines when arranged "below" the absorbent core layer 30. The electrodes 101-106 of the first plurality of electrodes 100 extend in a first direction L1, and the electrodes 201-206 of the second plurality of electrodes 200 extend in a second direction L2. The first L1 and second direction L2 are orthogonal/perpendicular according to the embodiment of Fig. 2A.

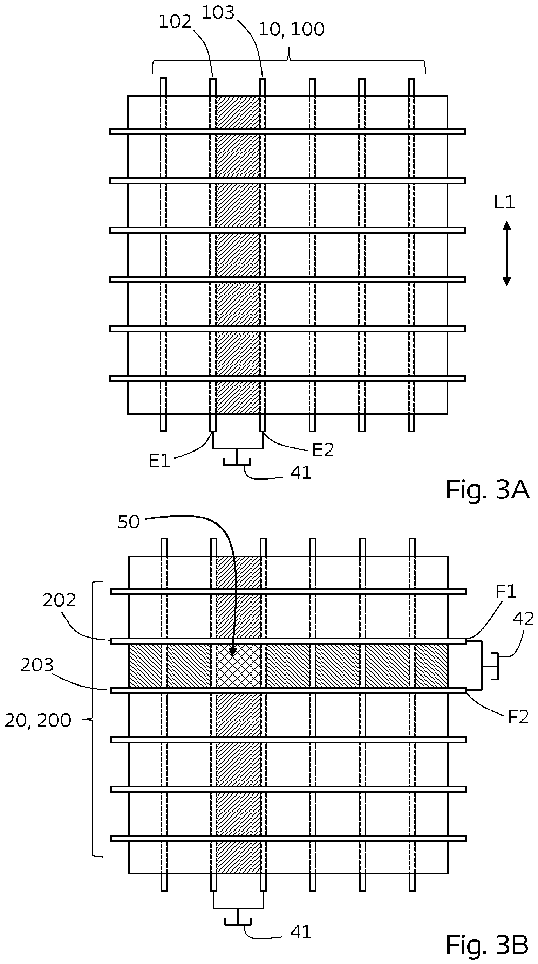

Each of the electrodes of the proximal 10 and distal electrode assembly 20 are connected to a monitor device configured to detect a short-circuiting event across a primary and a secondary electrode. For example, a primary and a secondary electrode can be two distinguishable electrodes of the first plurality of electrodes 100, or a primary and a secondary electrode can be two distinguishable electrodes of the second plurality of electrodes 200. This is discussed in greater detail in relation to Fig. 3A and Fig. 3B.

The cross-sectional view of Fig. 2B illustrates the first electrode 201 of the second plurality of electrodes 200 and the electrodes 101-106 of the first plurality of electrodes 100. The first plurality of electrodes 100 is arranged on the proximal side 31 of the absorbent core layer 30 and the second plurality of electrodes is arranged on the distal side 32 of the absorbent core layer 30. In

Fig. 2B, a liquid bridge 98, e.g. caused by exudate, is indicated by a box extending from the first electrode 101 to the second electrode 102 of the first plurality of electrodes 100. In this example, the primary electrode E1 is the first electrode 101, and the secondary electrode E2 is the second electrode 102 (or vice versa depending on definition). Thus, the liquid bridge 98 causes a short-circuiting event to occur across the primary electrode E1 and the secondary electrode E2 when a monitor device applying a voltage is connected to the wound dressing.

Fig. 3A illustrates a top view of the model of a wound dressing for a wound dressing system as shown in Fig. 1 and Fig. 2A. For simplicity, only relevant reference numbers are included.

Fig. 3A illustrates a case where a first short-circuiting event 41 has been detected across a primary electrode E1 and a secondary electrode E2 of the first plurality of electrodes 100, i.e. within the proximal electrode assembly 10. For example, the primary electrode E1 can be the second electrode 102 of the first plurality of electrodes 100, and secondary electrode E2 can be the third electrode 103 of the first plurality of electrodes 100. The first short-circuiting event 41 can be caused by exudate wetting the wound dressing and forming a liquid path between the primary E1 and secondary electrodes E2. Due to the arrangement of electrodes within the first plurality of electrodes 100, a monitor device connected to said plurality of electrodes can deduce the region/location of wetting to be within the hatched region, but not specifically where along the hatched region, i.e. where along the first direction L1.

A monitor device connected to the wound dressing, including connected to the first plurality of electrodes 100, and applying a voltage is configured to detect the first short-circuiting event 41.

Fig. 3B illustrates a case where a second short-circuiting event 42 has been detected across a primary electrode F1 and a secondary electrode F2 of the second plurality of electrodes 200, i.e. within the distal electrode assembly 20. For example, the primary electrode F1 can be the second electrode 202 of the second plurality of electrodes 200, and secondary electrode F2 can be the third electrode 203 of the second plurality of electrodes 200. The second short-circuiting event 42 can be caused by exudate having propagated through the absorbent core layer 30, i.e. after initially having caused the first short-circuiting event 41 as discussed in relation to Fig. 3A. Thus, the second short-circuiting event 42 is likely to occur after the first short-circuiting event 41.

A monitor device connected to the wound dressing, including connected to the second plurality of electrodes 200, and applying a voltage is configured to detect the second short-circuiting event 42.

Having detected a first short-circuiting event 41 and a second short-circuiting event 42, the connected monitor device is configured to determine a first region 50 where the primary E1 and secondary E2 electrodes of the first plurality of electrodes 100 and the primary F1 and secondary F2 electrodes of the second plurality of electrodes 200 overlap in the normal direction (cross-hatched area). In other words, the first region 50 is characterized by being bound by the primary electrode E1 and the secondary electrode E2 of the first plurality of electrodes 100, and by the primary electrode F1 and the secondary electrode F2 of the second plurality of electrodes 200, when seen in a direction normal to the appliance plane and hence the electrode assemblies. Thus, the first region 50 is a region of the wound dressing comprising short-circuited electrodes (E1, E2) of the first plurality of electrodes 100 and short-circuited electrodes (F1, F2) of the second plurality of electrodes 200. Based on the first region 50, the monitor device is configured to indicate an exudate uptake pattern of the wound dressing. In embodiments, the exudate uptake pattern is (in the vicinity of) the first region 50, as this first region 50 indicates the region where exudate is most likely to have occurred/wetted the wound dressing as electrodes of both the first plurality of electrodes 100 and the second plurality of electrodes 200 are short-circuited in this specific region.

The monitor device can be configured to determine a first time-delay between the short-circuiting event 41 across the primary electrode E1 and the secondary electrode E2 of the first plurality of electrodes 100 and the short-circuiting event 42 across the primary electrode F1 and the secondary electrode F2 of the second plurality of electrodes 200. The first time-delay can reflect the time it takes exudate to pass through the absorbent core layer, in embodiments where the proximal electrode assembly 10 is arranged on the proximal side of an absorbent core layer and the distal electrode assembly 20 is arranged on the distal side of the absorbent core layer. The monitor device can be configured to determine the exudate uptake pattern based on both the first region 50 and the first time-delay. For example, a large first time-delay can indicate a slow progression through the absorbent core layer and hence increases the probability that the wetting has spread further during the first time-delay (i.e., that the exudate, to a low degree of certainty, is still within the first region). On the contrary, a low first time-delay can indicate a fast progression through the absorbent core layer and hence indicates that the exudate is indeed (to high degree of certainty) still within the first region 50. The monitor device can be configured to output an accuracy/certainty of the indicated exudate uptake pattern based on the first region 50 and the first time-delay, where especially the latter is likely to affect the accuracy as described above.

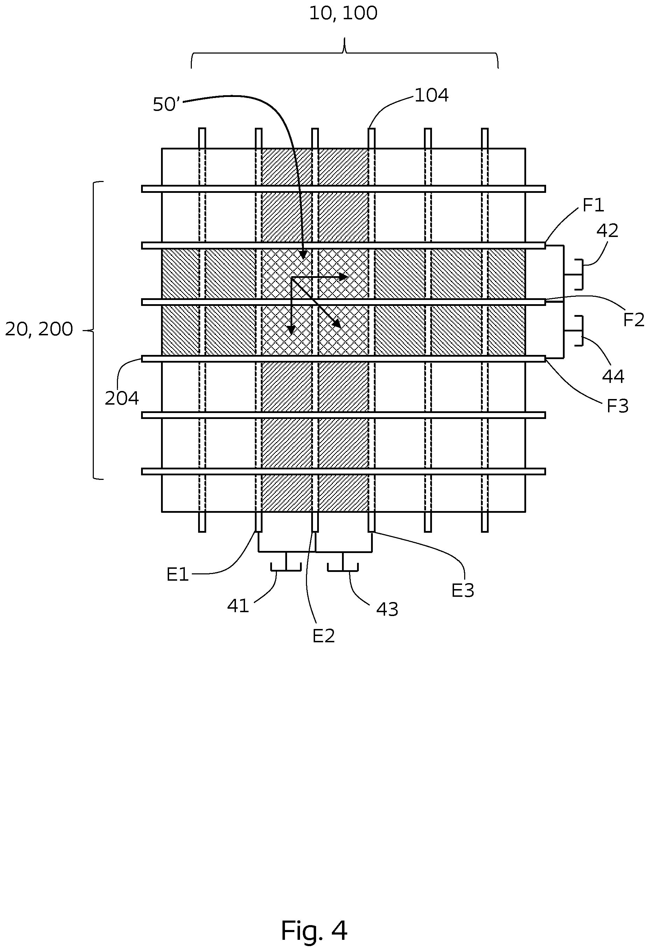

Fig. 4 illustrates a progression/spreading of the exudate uptake pattern as discussed in relation to Figs. 3A and 3B. The first region 50' (cross-hatched area) has now increased in area due to additional short-circuiting events having been detected by the monitor device. In particular, a third 43 and a fourth short-circuiting event 44 have been detected. The third short-circuiting event 43 was detected in the proximal electrode assembly 10 and the fourth short-circuiting event 44 was detected in the distal electrode assembly 20. The third short-circuiting event 43 was detected to be across the first/secondary electrode E1/E2 (now a joint electrode due to the preceding first short-circuiting event 41) and a tertiary electrode E3 (here, the fourth electrode 104 of the first plurality of electrodes 100). The fourth short-circuiting event 44 was detected to be across the first/secondary electrode F1/F2 (now a joint electrode due to the preceding second short-circuiting event 42) and a tertiary electrode F3 (here, the fourth electrode 204 of the second plurality of electrodes 200). The monitor device is configured to determine the first region 50' (now being larger in area compared to first region of Fig. 3B). Further, the monitor device is configured to indicate a propagation of the exudate uptake pattern as illustrated by the arrows within the first region 50'. The propagation of the exudate uptake pattern can be derived from the first region 50', as the first region 50' has increased in size in a certain direction based on where the third 43 and fourth short-circuiting event 44 occurred relative to the first 41 and second short-circuiting event 42.

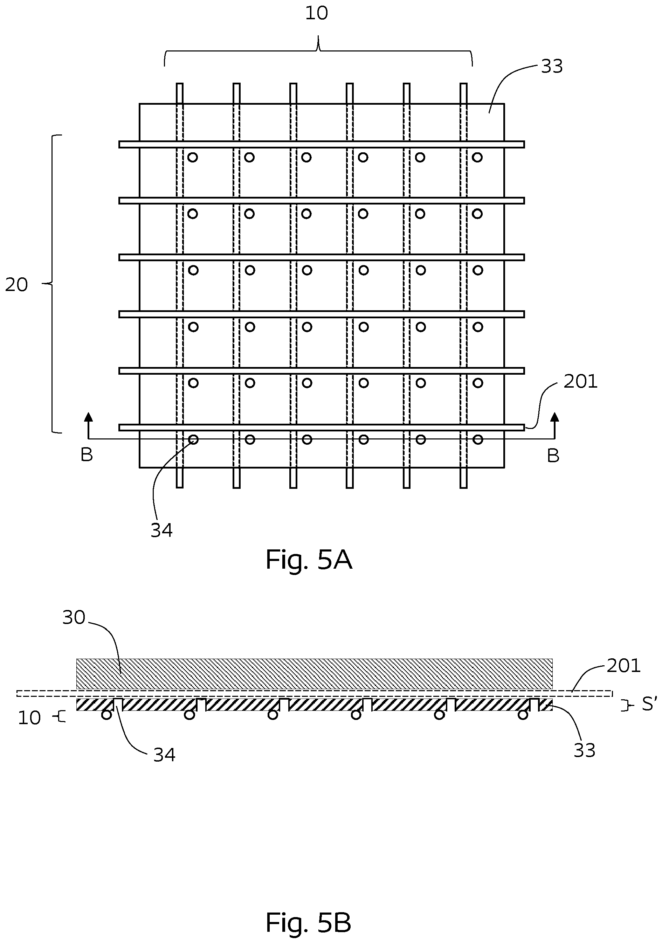

Fig. 5A illustrates a top view of a distal electrode assembly 20, a proximal electrode assembly 10, and a perforated spacer element 33 for use in a wound dressing for a wound dressing system similar to previously disclosed embodiments. Fig. 5B illustrates a cross-sectional view of the top view shown in Fig. 5A taken along the line B-B. The cross-sectional view further illustrates an absorbent core layer 30 and a first electrode 201 (dashed) of the second plurality of electrodes 200. The proximal electrode assembly 10 and the distal electrode assembly 20 are spaced by means of the spacer element 33. The spacer element 33 serves to separate the proximal electrode assembly 10 from the distal electrode assembly 20 by a first gap S'. Thus, in the embodiment, the first gap S is occupied by the spacer element 33. The spacer element 33 comprises through-going perforations/openings 34 allowing exudate to pass through the spacer element 33, i.e. allowing exudate to pass from the proximal 10 to the distal electrode assembly 20 and further into the absorbent core layer 30. The perforations 34 are arranged offset the electrodes, such that the risk of a liquid path (short-circuit) to form between/across an electrode of the proximal electrode assembly and an electrode of the distal electrode assembly is reduced. Adhesive can be provided on one or both sides of the spacer element 33, i.e. adhesive can be provided on a distal side of the spacer element and/or on a proximal side of the spacer element. The spacer element 33 can be non-absorbing thereby facilitating quick transfer of exudate from its proximal side to its distal side. Thereby, the first time-delay as previously described can be reduced (dependent on exudate production), increasing the accuracy of the indicated exudate uptake pattern when this is based on both the first region and the first time-delay. The electrodes of the proximal electrode assembly 10 and the electrodes of the distal electrode assembly 20 can be printed on each side of the spacer element 33.

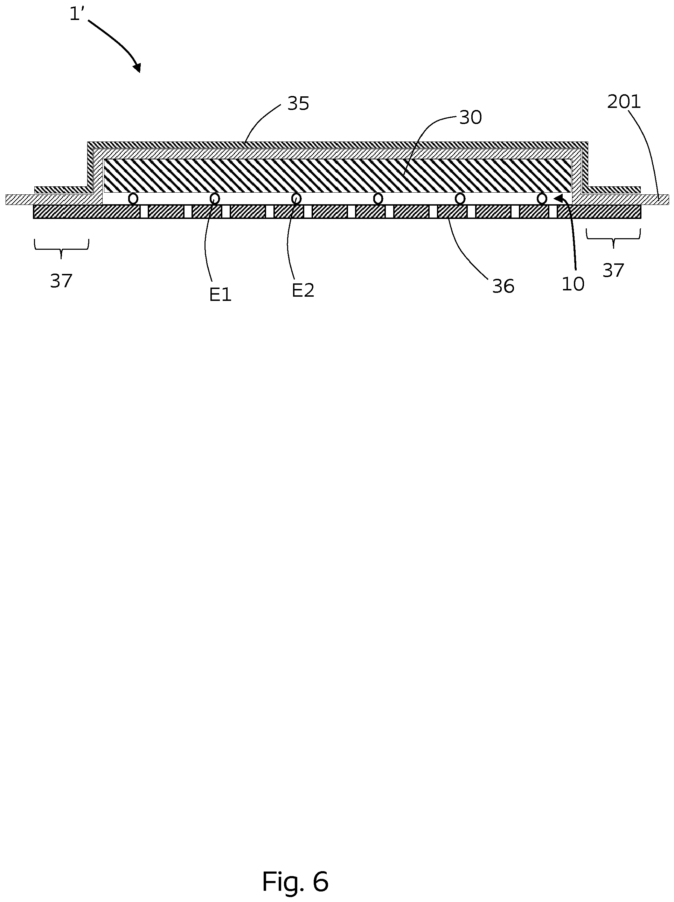

Fig. 6 illustrates a cross-sectional view of a wound dressing 1' for a wound dressing system. The wound dressing 1' comprises a top film 35, a distal electrode assembly (in this cross-sectional view illustrated by the first electrode 201), an absorbent core layer 30, a proximal electrode assembly 10, and a perforated first adhesive layer 36. The top film 35 and the perforated first adhesive layer 36 can be welded together along an edge portion 37 of the wound dressing 1', the welding allowing the electrodes to pass through, such as to form an interface for coupling to a monitor device. The top film prevents bacterial contamination as well as reduces stress and strain. The perforated first adhesive layer 36 is configured to adhere the wound dressing 1' to a skin surface of a user. The perforations of the perforated first adhesive layer 36 allow exudate from the wound to pass through the adhesive layer and thereby to be absorbed by the absorbent core layer 30. On the way towards the absorbent core layer 30, the exudate will pass by the proximal electrode assembly 10. Excessive amounts of exudate will eventually cause a liquid path to form between a primary electrode E1 and a secondary electrode E2 and thereby cause a short-circuiting event within the proximal electrode assembly 10. Such short-circuiting event is detectable by means of a monitor device applying a voltage and connected to the electrode assembly. The detection of a short-circuiting event is indicative of the presence of exudate in the region covered by the primary electrode E1 and the secondary electrode E2. Likewise, when exudate has been absorbed by the absorbent core layer 30, e.g. to a point where said absorbent core layer 30 is saturated, the exudate can cause a liquid path to form between a primary and secondary electrode of the distal electrode assembly, and in turn likewise cause a short-circuiting event. By comparing the electrodes - especially by comparing their relative position - involved in the short-circuiting event within the proximal electrode assembly and the electrodes involved in the short-circuiting event within the distal electrode assembly, a monitor device applying a voltage can determine a first region indicative of an exudate uptake pattern. Thus, based on the short-circuiting events, the monitor device can determine an exudate uptake pattern, the exudate uptake pattern being in the region of the short-circuiting events.

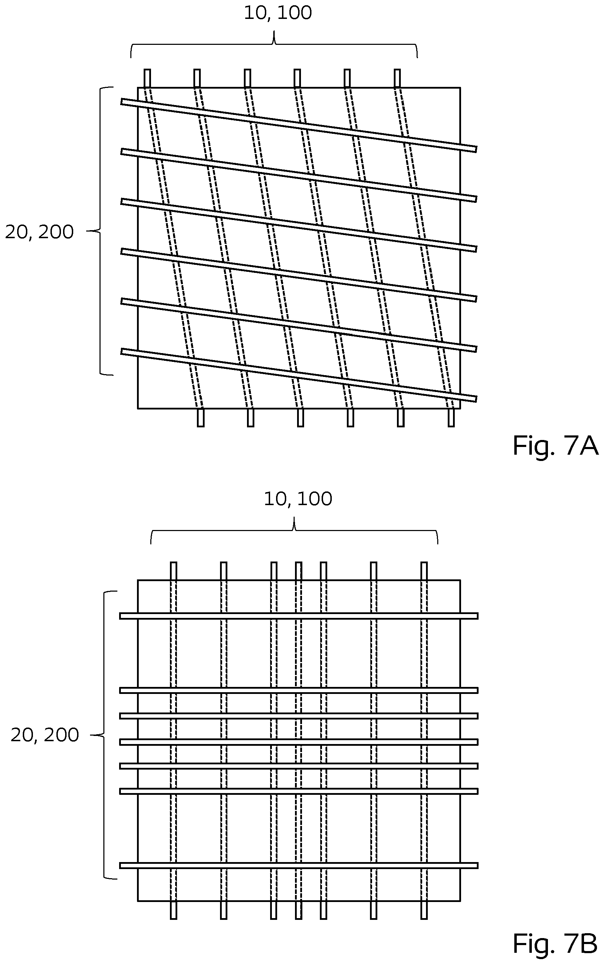

Figs. 7A and 7B illustrate alternative arrangements of electrodes of the proximal electrode assembly 10 and distal electrode assembly 20. In Fig. 7A, the first plurality of electrodes 100 are tilted relative to the second plurality of electrodes 200 at an angle different from 90 degrees. Such arrangement can be beneficial in alternative overall shapes of the wound dressing as such, e.g. in wound dressings not being rectangular, e.g. for triangular wound dressings and the like. Thus, by tilting/rotating the electrode assemblies relative to each other in oblique angles, certain design benefits can be achieved, e.g. in order to reduce complexity or increase sensor coverage in irregular shaped wound dressings. In Fig. 7B, varying distances between the electrodes of the electrode assemblies 10,20 are provided. Thereby, certain sensitivities can be achieved in different regions of the wound dressing. In Fig. 7B, a larger sensitivity in the centre region of the wound dressing is provided due to the relative larger number of electrodes 100,200 in this region relative to in the edge region of the wound dressing. In certain embodiments, non-parallel electrodes are foreseen, as well as non-straight electrodes, such as wavy electrodes.

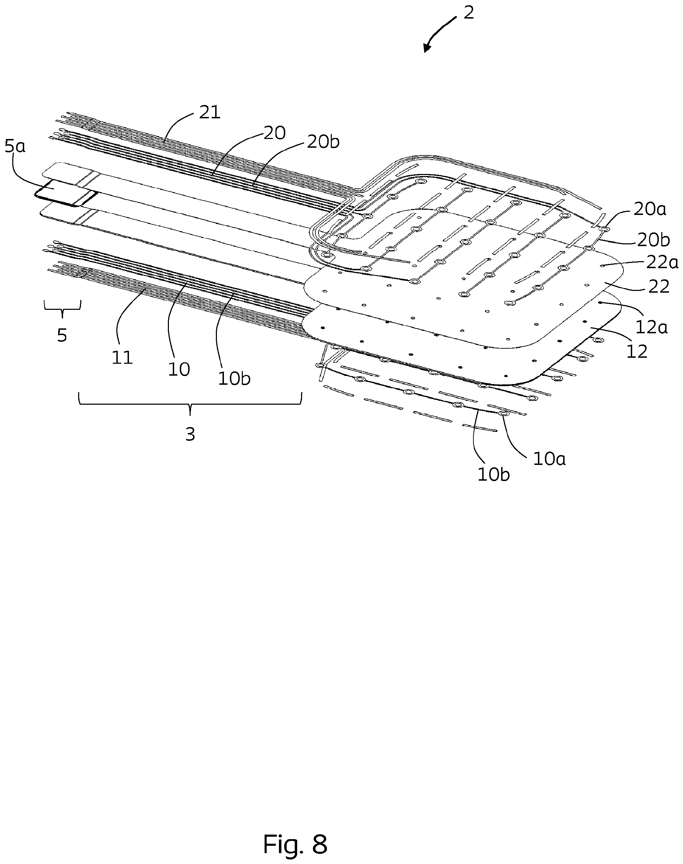

Fig. 8 illustrates an exploded view of a sensor pack 2 for a wound dressing of a wound dressing system. The sensor pack 2 is configured to be layered/aligned with a wound dressing, such as with an absorbent core layer of a wound dressing. The sensor pack 2 and an absorbent core layer in combination can, in embodiments, be considered the wound dressing according to the first aspect of the invention as disclosed in the detailed description. The following discussion provides a specific embodiment of a sensor pack suitable for use in a (conventional) wound dressing, such that such a wound dressing can be used in a wound dressing system as disclosed.

The sensor pack 2 comprises a distal masking layer 21, a distal electrode assembly 20, a distal (perforated) support layer 22, a proximal (perforated) support layer 12, a proximal electrode assembly 10, and a proximal masking layer 11. Further, the sensor pack 2 comprises a neck portion 3. Each of the aforementioned parts of the sensor pack 2 comprises a part of the neck portion 3. Further, the neck portion 3 comprises a reinforcement element 5a. The neck portion 3 terminates in a terminal interface 5 configured for connecting the electrodes of the sensor pack 2 with a monitor device comprising corresponding terminals.

The distal 22 and proximal support layer 12 double as a combined spacer element (reference number 33 in previous discussion). The support layers 12,22 provide a substrate for the electrode assemblies 10,20 when printing these as conductive traces. According to Fig. 8, two support layers 12,22 are provided. In alternative embodiments, a single support layer is provided, and the electrode assemblies can be printed on each respective side of such a single support layer. When providing two support layers 12,22, such support layers can be welded/glued together to form a single layer, which, according to embodiments, can function as the spacer element as previously discussed.

The masking layers 11,21 serve to define a series of sensing parts 10a,20a along each of the electrodes by insulating certain parts (conductor parts) 10b,20b of the electrodes. By insulating the conductor parts 10b, the risk of false signals is reduced. For example, the conductor parts 10b extend into the neck portion 3. In the neck portion 3, the electrodes of the proximal and distal electrode assemblies 10,20 are insulated by means of the masking layers 11,21. Thereby, the risk of generating signals pertaining to accidental wetting of the neck portion 3 is reduced. The support layers 12,22 are perforated as previously discussed for the spacer element. The perforations 12a,22a allow exudate to pass through the support layers 12,22 and reach the distal electrode assembly 20 and potentially an absorbent core layer arranged adjacent the sensor pack 2. According to Fig. 8, the perforations 12a,22a are holes aligned with sensing parts 10a,20a of the proximal electrode assembly 10 and the distal electrode assembly 20, respectively. The sensing parts 10a,20a encircle the perforations 12a,22a. Thereby is provided additional means for sensing exudate. As previously disclosed, the sensor pack 2 is adapted for detecting a short-circuiting event across a primary and secondary electrode of the proximal electrode assembly 10 (i.e., within said proximal electrode assembly 10) or the distal electrode assembly 20 (i.e., within said distal electrode assembly 20). By providing perforations 12a,22a aligned with the sensing parts 10a,20a, a short-circuiting event can be detected across a primary electrode of the proximal electrode assembly 10 and a primary electrode of the distal electrode assembly 20. Thus, such short-circuiting event is due to a liquid bridge/path extending from the proximal electrode assembly 10 to the distal electrode assembly 20 through one or more perforations 12a,22a. Such inter-electrode assembly short-circuiting event can potentially narrow down the first region as previously defined, as such first region can now be reduced to the region where a single primary electrode of the proximal electrode assembly 10 is short-circuited with a single primary electrode of the distal electrode assembly 20. Thus, the exudate uptake pattern can be narrowed down to the first region where the primary electrode of the proximal electrode assembly 10 intersects the primary electrode of the distal electrode assembly 20. The inter-electrode assembly short-circuiting event forms a joint electrode comprising the involved electrodes. The monitor device is configured for differentiating short-circuiting events and for comparing the short-circuiting electrodes both within an electrode assembly and across electrode assemblies in order to establish a first region and to indicate an exudate uptake pattern based on such first region. The sensor pack 2 is configured to be arranged adjacent an absorbent core layer of a wound dressing, e.g. on a proximal side of an absorbent core layer or on a distal side of an absorbent core layer, thereby facilitating an easy incorporation of sensors into the wound dressing. Thereby, the wound dressing can be assembled by layering the absorbent core layer and the sensor pack 2, and potentially a top film and a skin-friendly first adhesive layer for adhering the wound dressing to a skin surface of a user.

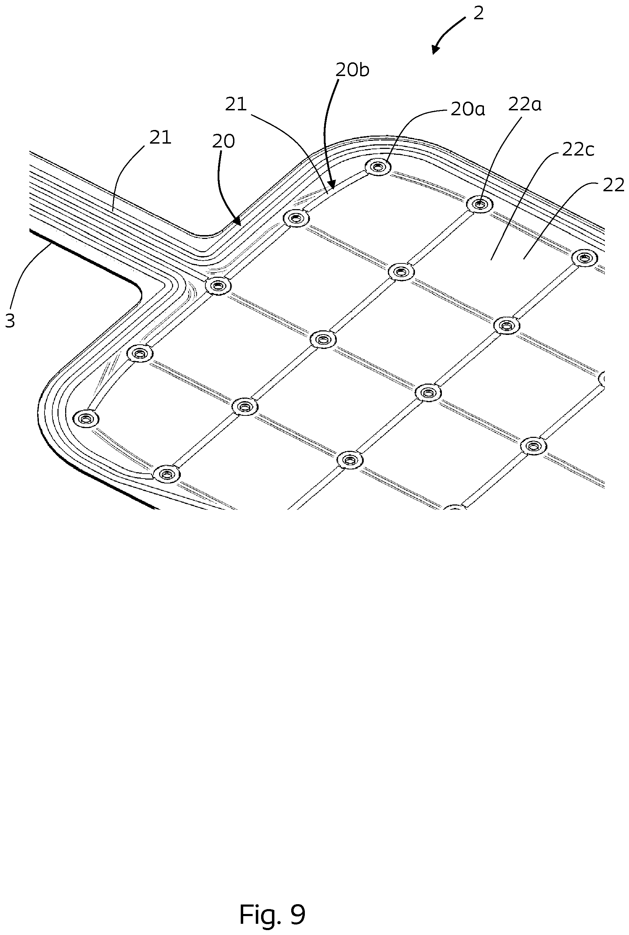

Fig. 9 illustrates a close-up view of the assembled sensor pack 2 of Fig. 8. The close-up view illustrates the distal electrode assembly 20, in particular the sensing parts 20a. Further, the distal masking layer 21 is shown to insulate conductor parts 20b of the distal electrode assembly 20. The perforations 22a (see Fig. 8 for perforations of proximal support layer) provides a passage for exudate to pass through the proximal (see Fig. 8) and the distal support layer 22. In an alternative embodiment, the perforations 22a are provided offset the sensing parts 20a, e.g. in the regions 22c of the support layer(s) 22 absent of electrodes - thereby, detection of inter-electrode assembly short-circuiting events is reduced, which can be desired in certain embodiments.

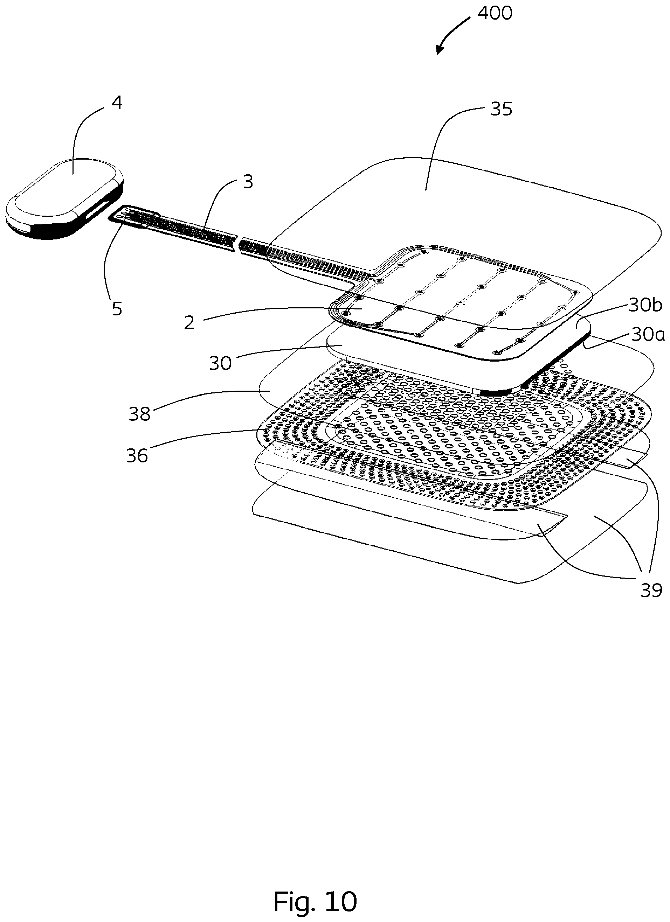

Fig. 10 illustrates an exploded view of a wound dressing system 400. The wound dressing system 400 comprises a monitor device 4 and a wound dressing 1. The wound dressing 1 comprises a sensor pack 2 as previously discussed, a top film 35, an absorbent core layer 30, an adhesive support layer 38, a first adhesive layer 36, and a release liner 39. The adhesive support layer 38 can be a polyurethane film. The release liner 39 can be a three-piece release liner as illustrated.

The sensor pack 2 is illustrated to be arranged on a distal side 30b of the absorbent core layer 30. In alternative embodiments, the sensor pack 2 is arranged on a proximal side 30a of the absorbent core layer 30. The monitor device 4 is releasably connectable to the terminal interface 5 of the neck portion 3 of the sensor pack 2. The monitor device 4 is configured to apply a potential difference across the electrodes of the electrode assemblies of the sensor pack 2 and to detect short-circuiting events, as well as other parameters, such as change in resistance and/or capacitance in the electrodes or across the electrodes, such as a change in resistance and/or capacitance due to moisture absorption in the absorbent core layer 30 or the support layers, if the support layers are absorbent to a certain degree. Short-circuiting events and additional data obtainable form the electrode assemblies are commonly denoted sensor data. The monitor device 4 is configured for collecting such sensor data through a first interface and to process such sensor data to determine the first region and/or the first time-delay as previously discussed.

The first adhesive layer 36 is configured for adhering the wound dressing 1 to the skin surface of a user once the protective release liner(s) 39 is removed. The first adhesive layer 36 and the adhesive support layer 38 are perforated for allowing exudate to pass through and reach the absorbent core layer 30.

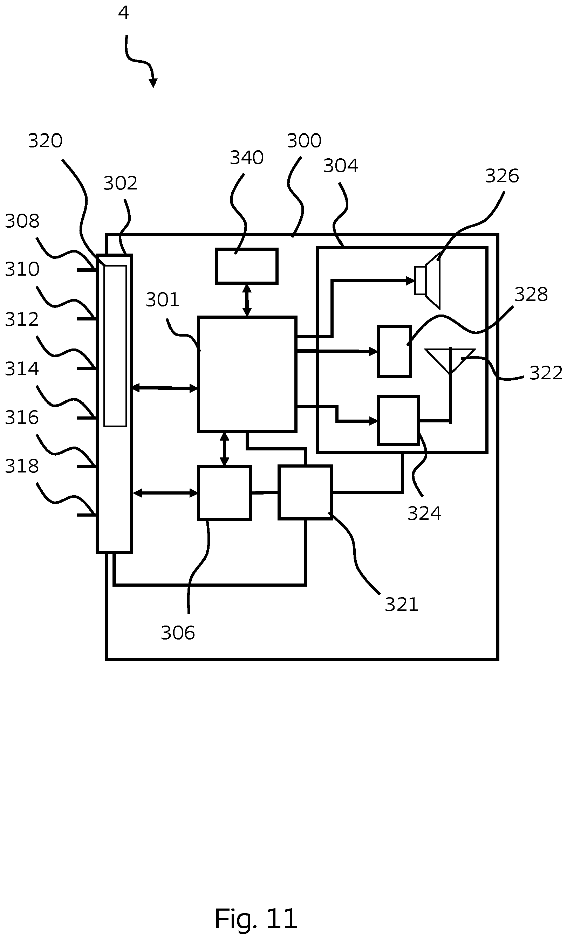

Fig. 11 illustrates a schematic block diagram of an exemplary monitor device 4. The monitor device 4 comprises a monitor device housing 300, a processor 301 and one or more interfaces, the one or more interfaces including a first interface 302 (appliance interface) and a second interface 304 (accessory interface). The monitor device 4 comprises a memory 306 for storing sensor data and/or parameter data based on the sensor data. The memory 306 is connected to the processor 301 and/or the first interface 302.

The first interface 302 is configured as an appliance interface for electrically and/or mechanically connecting the monitor device 4 to the wound dressing. The first interface 302 comprises a plurality of terminals 308,310,312,314,316,318 (or more or less, depending on the configuration of the wound dressing) for forming electrical connections with respective terminals of the wound dressing provided in the aforementioned terminal interface (reference number 5 in Figs. 8 and 10). The first interface 302 comprises a ground terminal 308, a first terminal 310, a second terminal 312 and a third terminal 314. The first interface 302 optionally comprises a fourth terminal 316 and a fifth terminal 318, and optionally a sixth terminal, and a seventh terminal. The first interface 302 of the monitor device 4 comprises a coupling part for forming a mechanical connection, such as a releasable coupling between the monitor device 4 and the neck portion of the wound dressing and/or sensor pack as previously discussed. The coupling part 320 and the terminals 308, 310, 312, 314, 316, and 318 of the first interface 302 may form (at least part of) a third coupling element of the monitor device 4.

The monitor device 4 comprises a power unit 321 for powering the monitor device 4 and active components thereof, i.e. the power unit 321 is connected to the processor 301, the first interface 302, the second interface 304, and memory 306. The power unit 321 comprises a battery and charging circuitry. The charging circuitry is connected to the battery and terminals of the first interface 302 for charging the battery via terminals of the first interface.

The second interface 304 of monitor device is configured as an accessory interface for connecting the monitor device 4 to one or more accessory devices such as a smartphone/-watch, a docking station, or a server. The second interface 304 comprises an antenna 322 and a wireless transceiver 324 configured for wireless communication with accessory device(s). Optionally, the second interface 304 comprises a loudspeaker 326 and/or a haptic feedback element 328 for provision of respective audio signal and/or haptic feedback to the user.

The monitor device 4 optionally comprises a sensor unit 340 connected to the processor 301. The sensor unit 340 can comprise a temperature sensor for feeding temperature data to the processor 301 and/or a G-sensor or accelerometer for feeding acceleration data to the processor 301.

The processor 301 is configured to apply a processing scheme, and the first interface 302 is configured for collecting sensor data from the wound dressing. The sensor data comprises first sensor data from a proximal electrode assembly and second sensor data from a distal electrode assembly. Optionally, the sensor data comprises third sensor data from a joint electrode formed by a short-circuited between the proximal electrode assembly and the distal electrode assembly. Optionally, the sensor data comprises additional sensor data. The sensor data can be stored in the memory 306 and/or processed in the processor 301 in order to obtain parameter data. The parameter data can be stored in the memory 306. The processor 301 is configured to apply a processing scheme, wherein to apply a processing scheme comprises obtain first parameter data based on the first sensor; obtain second parameter data based on the second sensor data; and optionally obtain third and/or additional parameter data based on third sensor data and/or additional sensor data. In other words, the processor 301 is configured to obtain first, second, and optionally third and additional parameter data based on respective first, second, and optionally third and additional sensor data. To apply a processing scheme comprises to determine a short-circuiting event, to determine a first region, or to indicate an exudate uptake pattern based on one or more, e.g. all, of the first parameter data, the second parameter data and potentially the third and additional parameter data, wherein the short-circuiting event is indicative of a wetting of the short-circuited electrodes due to presence of exudate. The monitor device 4 is configured to, in accordance with a determination of one or more, such as two, short-circuiting events, determine a first region, indicate an exudate uptake pattern, transmit a first monitor signal comprising monitor data indicative of the short-circuiting event, first region, and/or the exudate uptake pattern, via the second interface.

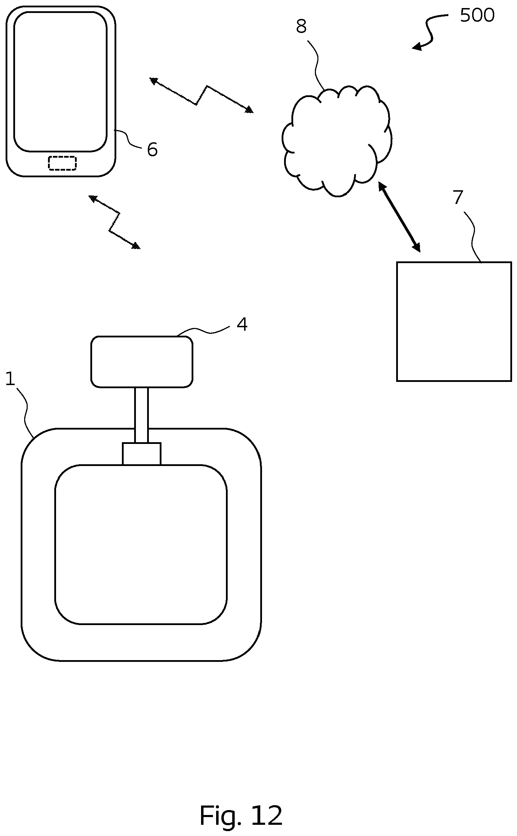

Fig. 12 illustrates an exemplary wound dressing system 500. The wound dressing system 500 comprises a wound dressing 1, a monitor device 4, and optionally an accessory device 6 (e.g. a mobile phone, such as a smartphone, or a smart watch). The monitor device 4 is connectable to the wound dressing 1 via respective interfaces of the monitor device 4 and wound dressing 1. The monitor device 4 is configured for wireless communication with the accessory device 6. Optionally, the accessory device 6 is configured to communicate with a server device 7 of the wound dressing system 500, e.g. via a network 8. The server device 7 may be operated and/or controlled by the wound dressing manufacturer and/or a service centre. Sensor data and/or parameter data based on the sensor data are obtained from electrodes/sensors of the wound dressing 1 by means of the monitor device 4. The monitor device 4 processes the sensor data and/or parameter data based on the sensor data to determine monitor data that are transmitted to the accessory device 6. In the illustrated wound dressing system 500, the accessory device 6 is a mobile phone, however the accessory device 6 may be embodied as another handheld device, such as a tablet device, or a wearable, such as a watch or other wrist-worn electronic device. Accordingly, the monitor device 4 is configured to determine and transmit monitor data to the accessory device 6.

## Claims

1. A wound dressing system (400, 500) configured to determine an exudate uptake pattern of a wound dressing (1), the wound dressing system (400, 500) comprising the wound dressing (1) extending in an appliance plane and comprising:
- an absorbent core layer (30) having a proximal side (31) and a distal side (32),
- a proximal electrode assembly (10) comprising a first plurality of electrodes (100), and
- a distal electrode assembly (20) comprising a second plurality of electrodes (200), the proximal electrode assembly (10) and the distal electrode assembly (20) being layered and spaced apart by a first material in a direction normal to the appliance plane; and
a monitor device (4) configured to detect a short-circuiting event across a primary electrode (E1) and a secondary electrode (E2) of the first plurality of electrodes (100) and a short-circuiting event across a primary electrode (F1) and a secondary electrode (F2) of the second plurality of electrodes (200); and
wherein the monitor device (4) is further configured to
- determine a first region (50) of the wound dressing (1), wherein the first region (50) is a region wherein the short-circuited primary electrode (E1) and secondary electrode (E2) of the first plurality of electrodes (100) and the short-circuited primary electrode (F1) and secondary electrode (F2) of the second plurality of electrodes (200) overlap in the direction normal to the appliance plane, and
- indicate an exudate uptake pattern of the wound dressing based on at least the first region (50).

2. The wound dressing system according to claim 1, wherein the monitor device is further configured to assign a first timestamp to the short-circuiting event across a primary (E1) electrode and a secondary electrode (E2) of the first plurality of electrodes (100) and to assign a second timestamp to the short-circuiting event across a primary electrode (F1) and a secondary electrode (F2) of the second plurality of electrodes (200), and to determine a first time-delay between said short-circuiting events based on said assigned first and second timestamps.

3. The wound dressing system according to claim 2, wherein the monitor device is configured to indicate the exudate uptake pattern of the wound dressing based on at least the first region and the first time-delay.

4. The wound dressing system according to any one of claims 1-3, wherein the electrodes of the first plurality of electrodes (100) are mutually parallel and extending in a first direction (L1), and wherein the electrodes of the second plurality of electrodes (200) are mutually parallel and extending in a second direction (L2), and wherein the first direction (L1) and the second direction (L2) are non-parallel.

5. The wound dressing system according to claim 4, wherein the first direction (L1) and the second direction (L2) are orthogonal.

6. The wound dressing system according to any one of claims 1-5, wherein the first material is at least a portion of the absorbent core layer (30).

7. The wound dressing system according to any one of claims 1-5, wherein the first material is a spacer element (33).

8. The wound dressing system according to any of claims 1-7, wherein the monitor device is further configured to detect one or more additional short-circuiting events across two or more electrodes of the first and/or second plurality of electrodes.

9. The wound dressing system according to claim 8, wherein the monitor device is further configured to indicate a propagation of the exudate uptake pattern of the wound dressing based on the detection of one or more additional short-circuiting events across two or more electrodes of the first and/or second plurality of electrodes.

10. The wound dressing system according to any of claims 1-9, wherein the wound dressing comprises a first adhesive layer arranged on the proximal side of the absorbent core layer, the first adhesive layer having a proximal surface configured for attachment of the wound dressing to a skin surface of a user.

11. The wound dressing system according to claim 10, wherein the first adhesive layer comprises perforations.

12. The wound dressing system according to any of claims 1-11, wherein the wound dressing comprises a monitor interface configured for electrically and/or mechanically connecting the wound dressing to the monitor device.

13. The wound dressing system according to any of claims 1-12, wherein the proximal electrode assembly (10) and the distal electrode assembly (20) are provided in a separate sensor pack (2) layered with the wound dressing (1), such as the absorbent core layer (30).

14. The wound dressing system according to any of claims 1-13, wherein the first material has a thickness between 30 µm and 100 µm.

15. The wound dressing system according to any of claims 1-13, wherein the first material is the absorbent core layer, and the thickness of the first material is between 0.8 mm and 10 mm.

## Patentansprüche

1. Wundverbandsystem (400, 500), das ausgestaltet ist, um ein Exsudataufnahmemuster eines Wundverbands (1) zu bestimmen, wobei das Wundverbandsystem (400, 500) den Wundverband (1) umfasst, der sich in einer Anwendungsebene erstreckt und folgendes umfasst:
- eine Absorbenskernschicht (30) mit einer proximalen Seite (31) und einer distalen Seite (32),
- eine proximale Elektrodenanordnung (10), umfassend eine erste Vielzahl von Elektroden (100), und
- eine distale Elektrodenanordnung (20), umfassend eine zweite Vielzahl von Elektroden (200), wobei die proximale Elektrodenanordnung (10) und die distale Elektrodenanordnung (20) geschichtet und durch ein erstes Material in einer Richtung senkrecht zu der Anwendungsebene beabstandet sind; und eine Überwachungsvorrichtung (4), die ausgestaltet ist, um ein Kurzschlussereignis über einer Primärelektrode (E1) und einer Sekundärelektrode (E2) der ersten Vielzahl von Elektroden (100) und ein Kurzschlussereignis über einer Primärelektrode (F1) und einer Sekundärelektrode (F2) der zweiten Vielzahl von Elektroden (200) zu detektieren; und wobei die Überwachungsvorrichtung (4) des Weiteren ausgestaltet ist zum:
- Bestimmen einer ersten Region (50) des Wundverbands (1), wobei die erste Region (50) eine Region ist, in der die kurzgeschlossene Primärelektrode (E1) und Sekundärelektrode (E2) der ersten Vielzahl von Elektroden (100) und die kurzgeschlossene Primärelektrode (F1) und Sekundärelektrode (F2) der zweiten Vielzahl von Elektroden (200) in der Richtung normal zu der Anwendungsebene überlappen, und
- Angeben eines Exsudataufnahmemusters des Wundverbands basierend auf mindestens der ersten Region (50) .

2. Wundverbandsystem nach Anspruch 1, wobei die Überwachungsvorrichtung des Weiteren ausgestaltet ist, um dem Kurzschlussereignis über einer Primärelektrode (E1) und einer Sekundärelektrode (E2) der ersten Vielzahl von Elektroden (100) einen ersten Zeitstempel zuzuordnen und dem Kurzschlussereignis über einer Primärelektrode (F1) und einer Sekundärelektrode (F2) der zweiten Vielzahl von Elektroden (200) einen zweiten Zeitstempel zuzuordnen, und um eine erste Zeitverzögerung zwischen den Kurzschlussereignissen basierend auf dem zugeordneten ersten und zweiten Zeitstempel zu bestimmen.

3. Wundverbandsystem nach Anspruch 2, wobei die Überwachungsvorrichtung ausgestaltet ist, um das Exsudataufnahmemuster des Wundverbands basierend auf mindestens der ersten Region und der ersten Zeitverzögerung anzugeben.

4. Wundverbandsystem nach einem der Ansprüche 1 bis 3, wobei die Elektroden der ersten Vielzahl von Elektroden (100) parallel zueinander sind und sich in eine erste Richtung (L1) erstrecken, und wobei die Elektroden der zweiten Vielzahl von Elektroden (200) parallel zueinander sind und sich in eine zweite Richtung (L2) erstrecken, und wobei die erste Richtung (L1) und die zweite Richtung (L2) nicht parallel sind.

5. Wundverbandsystem nach Anspruch 4, wobei die erste Richtung (L1) und die zweite Richtung (L2) orthogonal sind.

6. Wundverbandsystem nach einem der Ansprüche 1 bis 5, wobei das erste Material mindestens ein Anteil der Absorbenskernschicht (30) ist.

7. Wundverbandsystem nach einem der Ansprüche 1 bis 5, wobei das erste Material ein Abstandhalterelement (33) ist.

8. Wundverbandsystem nach einem der Ansprüche 1 bis 7, wobei die Überwachungsvorrichtung des Weiteren ausgestaltet ist, um ein oder mehrere zusätzliche Kurzschlussereignisse über zwei oder mehr Elektroden der ersten und/oder zweiten Vielzahl von Elektroden zu detektieren.

9. Wundverbandsystem nach Anspruch 8, wobei die Überwachungsvorrichtung des Weiteren ausgestaltet ist, um eine Ausbreitung des Exsudataufnahmemusters des Wundverbands basierend auf der Detektierung von einem oder mehreren zusätzlichen Kurzschlussereignissen über zwei oder mehr Elektroden der ersten und/oder zweiten Vielzahl von Elektroden anzugeben.

10. Wundverbandsystem nach einem der Ansprüche 1 bis 9, wobei der Wundverband eine erste Klebeschicht umfasst, die auf der proximalen Seite der Absorbenskernschicht angeordnet ist, wobei die erste Klebeschicht eine proximale Oberfläche aufweist, die zur Anbringung des Wundverbands auf einer Hautoberfläche eines Benutzers ausgestaltet ist.

11. Wundverbandsystem nach Anspruch 10, wobei die erste Klebeschicht Perforationen umfasst.

12. Wundverbandsystem nach einem der Ansprüche 1 bis 11, wobei der Wundverband eine Überwachungsschnittstelle umfasst, die zum elektrischen und/oder mechanischen Verbinden des Wundverbands mit der Überwachungsvorrichtung ausgestaltet ist.

13. Wundverbandsystem nach einem der Ansprüche 1 bis 12, wobei die proximale Elektrodenanordnung (10) und die distale Elektrodenanordnung (20) in einem separaten Sensorpack (2) geschichtet mit dem Wundverband (1), wie der Absorbenskernschicht (30), bereitgestellt werden.

14. Wundverbandsystem nach einem der Ansprüche 1 bis 13, wobei das erste Material eine Dicke zwischen 30 µm und 100 µm hat.

15. Wundverbandsystem nach einem der Ansprüche 1 bis 13, wobei das erste Material die Absorbenskernschicht ist, und die Dicke des ersten Materials zwischen 0,8 mm und 10 mm liegt.

## Revendications

1. Système de pansement (400, 500) configuré pour déterminer un modèle d'absorption d'exsudat d'un pansement (1), le système de pansement (400, 500) comprenant le pansement (1) s'étendant dans un plan d'appareil et comprenant :
- une couche centrale absorbante (30) présentant une face proximale (31) et une face distale (32),
- un ensemble d'électrodes proximales (10) comprenant une première pluralité d'électrodes (100), et
- un ensemble d'électrodes distales (20) comprenant une seconde pluralité d'électrodes (200), l'ensemble d'électrodes proximales (10) et l'ensemble d'électrodes distales (20) étant superposés et espacés par un premier matériau dans une direction normale au plan de l'appareil ; et
un dispositif de surveillance (4) configuré pour détecter un événement de court-circuit entre une électrode primaire (E1) et une électrode secondaire (E2) de la première pluralité d'électrodes (100) et un événement de court-circuit entre une électrode primaire (F1) et une électrode secondaire (F2) de la seconde pluralité d'électrodes (200) ; et
dans lequel le dispositif de surveillance (4) est en outre configuré pour
- déterminer une première région (50) du pansement (1), dans lequel la première région (50) est une région dans laquelle l'électrode primaire (E1) et l'électrode secondaire (E2) court-circuitées de la première pluralité d'électrodes (100) et l'électrode primaire (F1) et l'électrode secondaire (F2) court-circuitées de la seconde pluralité d'électrodes (200) se chevauchent dans la direction normale au plan de l'appareil, et
- indiquer un modèle d'absorption d'exsudat du pansement sur la base d'au moins la première région (50).

2. Système de pansement selon la revendication 1, dans lequel le dispositif de surveillance est en outre configuré pour attribuer une première estampille temporelle à l'événement de court-circuit entre une électrode primaire (E1) et une électrode secondaire (E2) de la première pluralité d'électrodes (100) et pour attribuer une seconde estampille temporelle à l'événement de court-circuit entre une électrode primaire (F1) et une électrode secondaire (F2) de la seconde pluralité d'électrodes (200), et pour déterminer un premier retard entre lesdits événements de court-circuit sur la base desdites première et seconde estampilles temporelles attribuées.

3. Système de pansement selon la revendication 2, dans lequel le dispositif de surveillance est configuré pour indiquer le modèle d'absorption d'exsudat du pansement sur la base d'au moins la première région et le premier retard.

4. Système de pansement selon l'une quelconque des revendications 1 à 3, dans lequel les électrodes de la première pluralité d'électrodes (100) sont mutuellement parallèles et s'étendent dans une première direction (L1), et dans lequel les électrodes de la seconde pluralité d'électrodes (200) sont mutuellement parallèles et s'étendent dans une seconde direction (L2), et dans lequel la première direction (L1) et la seconde direction (L2) ne sont pas parallèles.

5. Système de pansement selon la revendication 4, dans lequel la première direction (L1) et la seconde direction (L2) sont orthogonales.

6. Système de pansement selon l'une quelconque des revendications 1 à 5, dans lequel le premier matériau est au moins une partie de la couche centrale absorbante (30).

7. Système de pansement selon l'une quelconque des revendications 1 à 5, dans lequel le premier matériau est un élément d'espacement (33).

8. Système de pansement selon l'une quelconque des revendications 1 à 7, dans lequel le dispositif de surveillance est en outre configuré pour détecter un ou plusieurs événements de court-circuit supplémentaires entre deux électrodes ou plus de la première et/ou de la seconde pluralité d'électrodes.

9. Système de pansement selon la revendication 8, dans lequel le dispositif de surveillance est en outre configuré pour indiquer une propagation du modèle d'absorption d'exsudat du pansement en fonction de la détection d'un ou plusieurs événements de court-circuit supplémentaires entre deux électrodes ou plus de la première et/ou de la seconde pluralité d'électrodes.

10. Système de pansement selon l'une quelconque des revendications 1 à 9, dans lequel le pansement comprend une première couche adhésive agencée sur la face proximale de la couche centrale absorbante, la première couche adhésive ayant une surface proximale configurée pour la fixation du pansement à la surface cutanée d'un utilisateur.

11. Système de pansement selon la revendication 10, dans lequel la première couche adhésive comprend des perforations.

12. Système de pansement selon l'une quelconque des revendications 1 à 11, dans lequel le pansement comprend une interface de moniteur configurée pour connecter électriquement et/ou mécaniquement le pansement au dispositif de moniteur.

13. Système de pansement selon l'une quelconque des revendications 1 à 12, dans lequel l'ensemble d'électrodes proximales (10) et l'ensemble d'électrodes distales (20) sont fournis dans un bloc de capteurs (2) distinct déposé en couche avec le pansement (1), tel que la couche centrale absorbante (30).

14. Système de pansement selon l'une quelconque des revendications 1 à 13, dans lequel le premier matériau a une épaisseur entre 30 µm et 100 µm.

15. Système de pansement selon l'une quelconque des revendications 1 à 13, dans lequel le premier matériau est la couche centrale absorbante, et l'épaisseur du premier matériau est entre 0,8 mm et 10 mm.
